# EUROPEAN PATENT SPECIFICATION

(11) **EP 2 194 143 B1**
(45) Date of publication and mention of the grant of the patent: **29.06.2016**
(21) Application number: 10150772.1
(22) Date of filing: 02.05.2007
(51) Int. Cl.: C12Q 1/68

(54) **Phenotype prediction**
Phänotyp-Vorhersage
Prédiction de phénotypes

(30) Priority: 02.05.2006 NZ 54695306; 19.03.2007 US 919104 P
(43) Date of publication of application: 09.06.2010
(62) Divisional of application: 07733650.1
(73) Proprietor: University of Southampton, Southampton, Hampshire SO17 IBJ (GB); Auckland Uniservices Limited, Auckland (NZ)
(72) Inventor: Godfrey, Keith, Malcolm, Ashurst, Hampshire SO40 7AJ (GB); Hanson, Mark, Adrian, Hursley, Hampshire SO21 2JY (GB); Burdge, Graham Charles, Eastleigh, Hampshire SO53 4LE (GB); Lillycrop, Karen Ann, Chichester, PO20 1LY (GB); Cooper, Cyrus, Southampton, Hampshire SO16 7HW (GB); Gluckman, Peter David, Auckland (NZ); Vickers, Mark Hedley, Auckland (NZ)
(74) Representative: Lord, Hilton David

(56) References cited:
- WO-A-2004/067777
- WO-A-2006/034879
- WO-A2-01/77375
- ZESCHNIGK M ET AL: "A SINGLE-TUBE PCR TEST FOR THE DIAGNOSIS OF ANGELMAN AND PRADER-WILLI SYNDROME BASED ON ALLELIC METHYLATION DIFFERENCES AT THE SNRPN LOCUS" EUROPEAN JOURNAL OF HUMAN GENETICS, KARGER, BASEL, CH, vol. 5, no. 2, 1997, pages 94-98, XP009011533 ISSN: 1018-4813
- MORGAN H D ET AL: "Epigenetic inheritance at the agouti locus in the mouse." NATURE GENETICS NOV 1999, vol. 23, no. 3, November 1999 (1999-11), pages 314-318, XP002458503 ISSN: 1061-4036
- WATERLAND R A ET AL: "Early nutrition, epigenetic changes at transposons and imprinted genes, and enhanced susceptibility to adult chronic diseases" NUTRITION 2004 UNITED STATES, vol. 20, no. 1, 2004, pages 63-68, XP002458504 ISSN: 0899-9007
- MACLENNAN N K ET AL: "Uteroplacental insufficiency alters DNA methylation, one-carbon metabolism, and histone acetylation in IUGR rats" PHYSIOLOGICAL GENOMICS 2004 UNITED STATES, vol. 18, 2004, pages 43-50, XP002458505 ISSN: 1531-2267
- LILLYCROP KAREN A ET AL: "Dietary protein restriction of pregnant rats induces and folic acid supplementation prevents epigenetic modification of hepatic gene expression in the offspring." THE JOURNAL OF NUTRITION JUN 2005, vol. 135, no. 6, June 2005 (2005-06), pages 1382-1386, XP002458506 ISSN: 0022-3166
- REES W D ET AL: "Maternal protein deficiency causes hypermethylation of DNA in the livers of rat fetuses." THE JOURNAL OF NUTRITION JUL 2000, vol. 130, no. 7, July 2000 (2000-07), pages 1821-1826, XP002458507 ISSN: 0022-3166
- HORSTHEMKE BERNHARD ET AL: "Assisted reproduction: the epigenetic perspective." HUMAN REPRODUCTION UPDATE 2005 SEP-OCT, vol. 11, no. 5, September 2005 (2005-09), pages 473-482, XP002458508 ISSN: 1355-4786
- GILLESSEN-KAESBACH GABRIELE ET AL: "A previously unrecognised phenotype characterised by obesity, muscular hypotonia, and ability to speak in patients with Angelman syndrome caused by an imprinting defect" EUROPEAN JOURNAL OF HUMAN GENETICS, vol. 7, no. 6, September 1999 (1999-09), pages 638-644, XP002458509 ISSN: 1018-4813
- WU Q ET AL: "Parental obesity and overweight affect the body-fat accumulation in the offspring: The possible effect of a high-fat diet through epigenetic inheritance" OBESITY REVIEWS 2006 UNITED KINGDOM, vol. 7, no. 2, 21 April 2006 (2006-04-21), pages 201-208, XP002458510
- RASSOULZADEGAN MINOO ET AL: "Transvection effects involving DNA methylation during meiosis in the mouse." THE EMBO JOURNAL 1 FEB 2002 LNKD- PUBMED:11823436, vol. 21, no. 3, 1 February 2002 (2002-02-01), pages 440-450, XP002580991 ISSN: 0261-4189
- K. M. GODFREY ET AL: 'Epigenetic Gene Promoter Methylation at Birth Is Associated With Child's Later Adiposity' DIABETES vol. 60, no. 5, 01 May 2011, pages 1528 - 1534, XP055161964 DOI: 10.2337/db10-0979 ISSN: 0012-1797
- MAHUA CHOUDHURY ET AL: 'Obesity: Childhood obesity-methylate now, pay later?' NATURE REVIEWS ENDOCRINOLOGY vol. 7, no. 8, 01 August 2011, pages 439 - 440, XP055161268 DOI: 10.1038/nrendo.2011.102 ISSN: 1759-5029

## Description

### Field of the Invention

The invention in general terms relates to the use of epigenetic markers in perinatal tissues as a means for predicting the propensity for the occurrence of a phenotype in an individual. In particular, for example, the invention relates to the prediction of a propensity for altered body composition, and altered bone mineral content occurring in an individual.

### Background Art

The term 'epigenetic' is used to refer to structural changes to genes that do not alter the nucleotide sequence. Of particular relevance is methylation of specific CpG dinucleotides in gene promoters and alterations in DNA packaging arising from chemical modifications of the chromatin histone core around which DNA wraps. Such epigenetic inheritance systems can be random with respect to the environment and have been termed epimutations, or specific epigenetic changes can be induced by the environment. DNA methylation is established during early development and persists into adulthood. The inventors have, however, now for the first time obtained data linking epigenetic change, more particularly degree of gene methylation, in perinatal tissues to phenotypic characteristics in later life

Epidemiological observations previously implicated early development in the etiology of common chronic diseases, including cardiovascular disease, type-2 diabetes, obesity, metabolic syndrome, non-alcoholic steatohepatitis, impaired skeletal growth, osteoporosis, chronic obstructive airways disease including asthma, susceptibility to infection, mental illness and affective disorder, impaired cognition, cancer, impaired renal function, reproductive health and auto-immune disorders. In humans, nutritional constraint before birth has been shown to be associated with increased risk of metabolic syndrome and cardiovascular disease (Godfrey & Barker (2001); Public Health Nutrition, 4, 611-624). This causal association has been replicated in animal models of nutritional constraint during pregnancy. The phenomenon may involve adaptations to fetal physiology which predict an unfavourable postnatal environment, such as limited nutrient availability, and so improve survival. However, if nutrients are abundant, the offspring may be less able to adapt to abundant nutrient supply which can ultimately result in disease (Gluckman & Hanson (2004) Science, 305, 1733-1736). In humans, a period of nutritional constraint during pregnancy can determine the risk of developing obesity in late middle age (Ravelli et al. (1999) Am. J. Clin. Nutr. 70, 811-816).

More recently it has been observed that maternal protein-restriction during rat pregnancy induces an alteration in the methylation of the glucocortioid receptor (GR) promoter associated with altered expression of the receptor in the liver of juvenile offspring (Lillycrop et al. (2005) J. Nutr. 135(6) 1382-1386). However, this observed epigenetic change associated with changed expression does not express itself as obesity. Alteration in the methylation of the GR promoter has therefore not been considered as a relevant epigenetic change in the treatment of obesity. Further, those knowledgeable in the art have previously considered that epigenetic change is only of relevance when a direct and reciprocal change in expression in the relevant gene is also observed (as in the case of Lillycrop et al. 2005). The evaluation of epigenetic change independent of changes in gene expression has not previously been considered of value and has not been investigated. The basis of this invention is the finding that epigenetic changes in themselves are of prognostic and diagnostic value.

That degree of gene methylation can be linked to propensity for a phenotypic characteristic was first recognised by the inventors as a result of rat studies linking altered methylation of the GR promoter in rat pup adipose tissue with maternal nutritional status and pup body weight on a high fat diet. Subsequent studies reported herein have confirmed that, equally, degree of methylation of specific genes in human perinatal tissue, e.g. umbilical cord, can be linked to a variety of future phenotypic characteristics including but not limited to characteristics of body composition/growth (total and /or proportionate body fat mass, total and /or proportionate lean body mass, bone mineral content or density and height), cognitive development (intellectual quotient (IQ)), neuro-behavioural status (e.g. hyperactivity) and cardiovascular structure and function (e.g. blood pressure, aortic compliance, left ventricular mass and coronary artery diameter). For the prediction of phenotypes, epigenetic markers may be used individually and in combinations. Such use of epigenetic markers may be of particular use not only in relation to managing propensity for occurrence of undesirable phenotypic characteristics in humans, e.g. obesity, but also, for example, in enabling economic decisions on future production characteristics in agricultural animals. Such use of epigenetic markers enables the targeting of corrective strategies where propensity for an undesirable phenotypic characteristic is identified.

### Summary of the invention

Disclosed herein is a method of a predicting a phenotypic characteristic of a human or non-human animal which comprises determining the degree of an epigenetic alteration of a gene or a combination of genes in a tissue, wherein the degree of said epigenetic alteration of the gene or genes of interest correlates with propensity for said phenotypic characteristic.

In one aspect of the present invention there is provided a method of predicting a phenotypic characteristic of a human or non-human animal, said phenotypic characteristic being selected from:
(i) total body lean mass and/or percentage lean mass, or susceptibility for sarcopenia or impaired muscle function;
(ii) impaired skeletal development associated with reduced bone mineral content or a disease condition associated with low bone mineral content such as osteoporosis;
and said method comprising determining the degree of methylation of the promoter of the Retinoid X receptor, alpha (RXRA) gene or a combination of the promoter of the RXRA gene and at least one other gene in a perinatal tissue sample, wherein the degree of said methylation of the gene or genes of interest correlates with propensity for said phenotypic characteristic.

As indicated above, the epigenetic alteration determined is gene promoter methylation, and the tissue is a perinatal tissue sample containing genomic DNA of the individual of interest taken prior to, at, or soon after birth (generally in the case of human neonates within a month of birth), preferably for example, an umbilical cord sample. However, other tissue may prove useful including in addition to adipose tissue, blood (including fetal and cord blood), placenta, chorionic villus biopsy, amniotic fluid, hair follicles, buccal smears and muscle biopsies. Preferably such tissue will be from a newborn or taken from an infant within a few weeks of birth, more preferably within a few days of birth (less than a week), although samples taken much later, e.g. within about 6 months or longer may prove useful. As indicated above, adipose tissue may be a suitable choice particularly, for example, if the phenotypic characteristic of interest is obesity.

Also disclosed herein is a method of predicting the propensity for obesity in an individual including the step of testing for altered methylation of the gene encoding the glucocorticoid receptor (GR). However, other genes may be preferred for methylation determination for this purpose. For example, one or more genes may be chosen for which association has been identified between degree of promoter methylation and total and /or proportionate body fat mass. The gene or genes may be selected from any of those identified in the exemplification as exhibiting such association in 9 year old children relying on umbilical cord samples, especially for example the endothelial nitric oxide synthase (NOS3) gene, the matrix metalloproteinase-2 (MMP2) gene, the phosphoinositide-3-kinase, catalytic, δ-polypeptide (P13KCD) gene and the heparan-α-glucosaminide N-acetyltransferase (HGSNAT) gene.

Preferably, for example, alteration of methylation is tested for in the adipose tissue of the individual or in an umbilical cord sample obtained at or soon after birth.

Preferably the individual is a neonate, infant, child or young adult.

Also disclosed herein is a method of managing incidence of obesity in an individual including the steps of:
(1) testing the individual for altered methylation of the glucocorticoid receptor gene or one or more other genes as discussed above; and
(2) managing the diet, behaviour or physical activity of the individual if the individual exhibits altered methylation of said gene or genes.

Also disclosed herein is a method of managing the incidence of obesity in a population including the steps of:
(1) screening individuals from the population for altered methylation of the glucocorticoid receptor gene or one or more other genes as discussed above; and
(2) changing the diet, behaviour or physical activity of the individuals in the population that exhibit altered methylation of said gene or genes.

Further aspects of the invention will be apparent from the more detailed description and examples below and with reference to the figures, which are now detailed.

### Brief description of figures

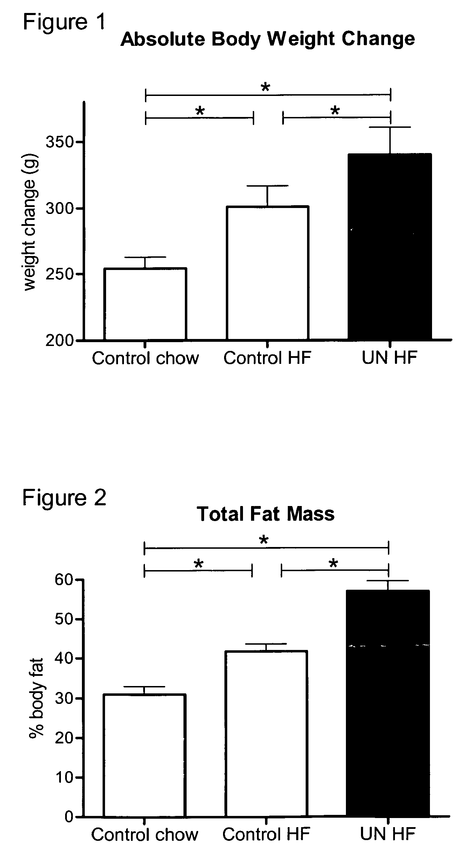

Figure 1 shows from the rat studies noted above and further described in Reference Example 1 absolute body weight change from weaning until day 170; control denotes offspring from mothers fed ad-libitum throughout pregnancy, UN denotes offspring from mothers fed at 30% of ad-libitum throughout pregnancy, chow denotes normal rodent diet, HF denotes high fat diet (45% kcals as fat). N=8 per group, data are mean ± SEM.
Figure 2 shows from the same studies total body fat mass (% total body fat) at day 170 as quantified by dual energy x-ray absorbtiometry (DEXA) N=8 per group, data are mean ± SEM.

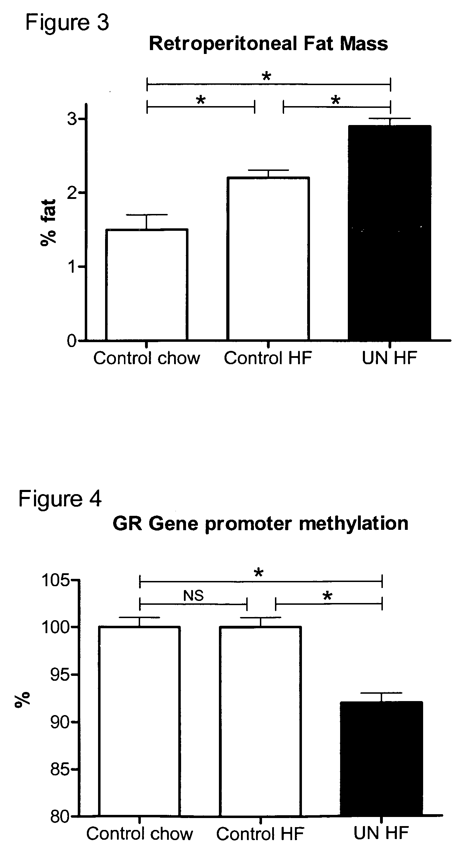

Figure 3 shows from the same studies retroperitoneal fat depot mass expressed relative to body weight. N=8 per group, data are mean ± SEM.
Figure 4 shows from the same studies glucocorticoid receptor (GR) gene promoter methylation expressed as a percentage relative to chow fed controls for progeny fed a high fat diet, N=6-8 per group, data are mean ± SEM.

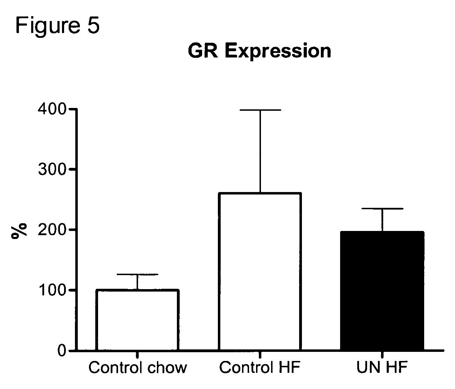

Figure 5 shows from the same studies glucocorticoid receptor gene expression expressed as a percentage relative to chow fed controls for progeny fed a high fat diet, N=6-8 per group, data are mean ±SEM.

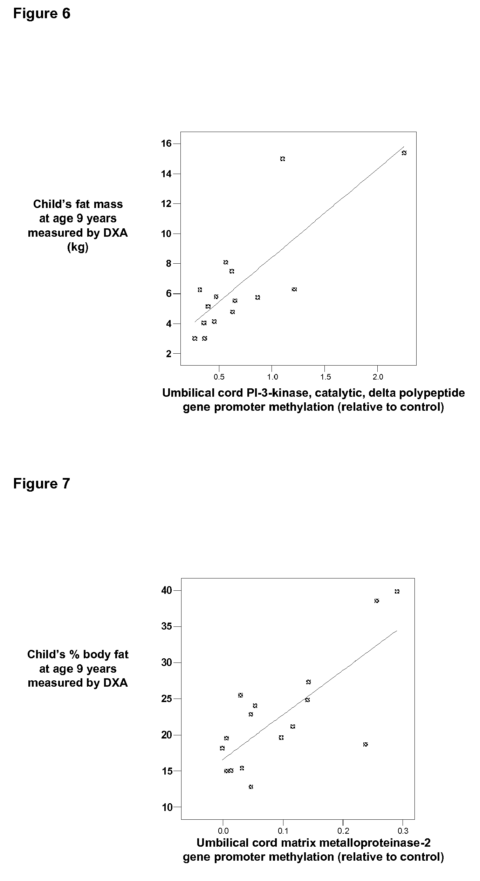

Figure 6 shows from the human studies described in Example 1, umbilical cord PI3KCD (PI-3-kinase, catalytic, delta polypeptide) gene promoter methylation (relative to control) in relation to the child's fat mass at age nine years measured by dual energy X-ray absorptiometry (DXA). N=15; r=0.68, P=0.005 by Spearman correlation.
Figure 7 shows from the human studies described in Example 1, umbilical cord MMP2 (matrix metalloproteinase-2 gene) promoter methylation (relative to control) in relation to the child's proportionate fat mass at age nine years measured by dual energy X-ray absorptiometry (DXA). N=16; r=0.66, P=0.005 by Spearman correlation.

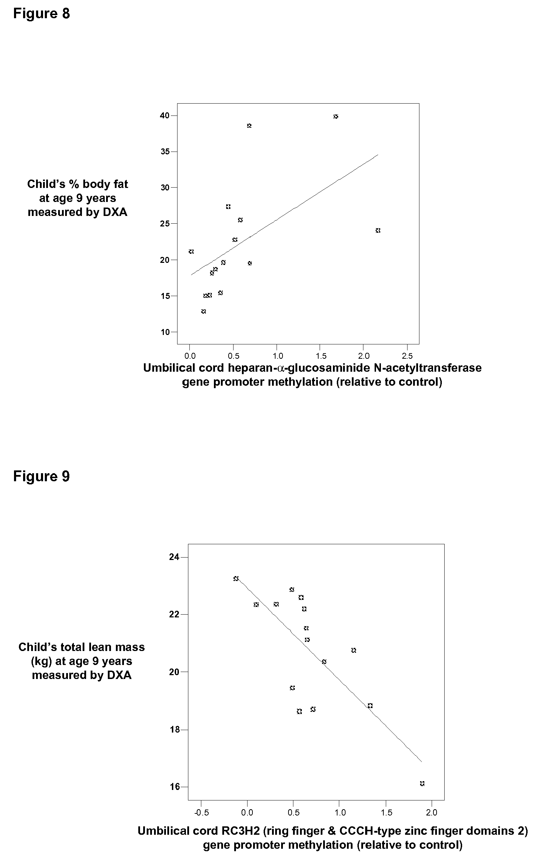

Figure 8 shows from the human studies described in Example 1, umbilical cord HGSNAT (heparan-α-glucosaminide N-acetyltransferase) gene promoter methylation (relative to control) in relation to the child's proportionate fat mass at age nine years measured by dual energy X-ray absorptiometry (DXA). N=15; r=0.73, P=0.002 by Spearman correlation.
Figure 9 shows from the human studies described in Example 1, umbilical cord RC3H2 (ring finger & CCCH-type zinc finger domains 2) gene promoter methylation (relative to control) in relation to the child's total lean mass at age nine years measured by dual energy X-ray absorptiometry (DXA). N=15; r=-0.70, P=0.004 by Spearman correlation.

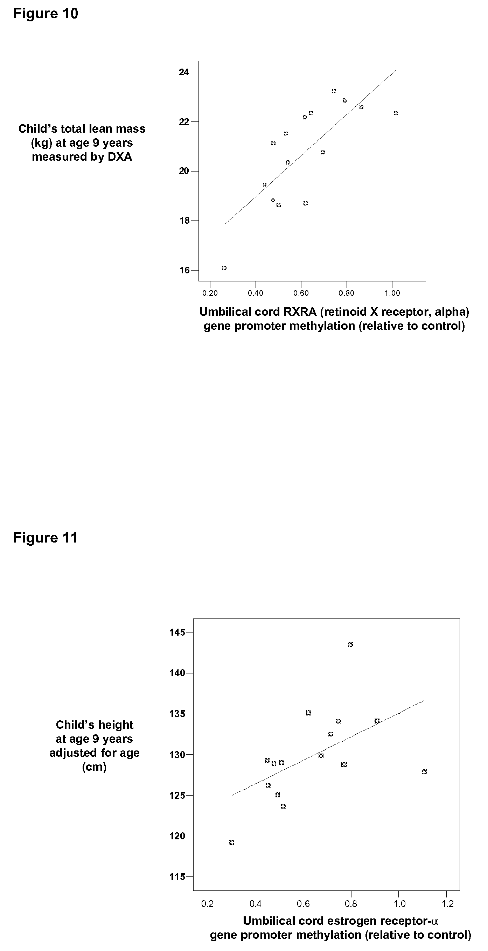

Figure 10 shows from the human studies described in Example 1, umbilical cord RXRA (retinoid X receptor, alpha) gene promoter methylation (relative to control) in relation to the child's total lean mass at age nine years measured by dual energy X-ray absorptiometry (DXA). N=15; r=0.76, P=0.001 by Spearman correlation.
Figure 11 shows from the human studies described in Example 1, umbilical cord ESR1 (estrogen receptor-α) gene promoter methylation (relative to control) in relation to the child's height at age nine years. N=15; r=0.51, P=0.05 by Spearman correlation.

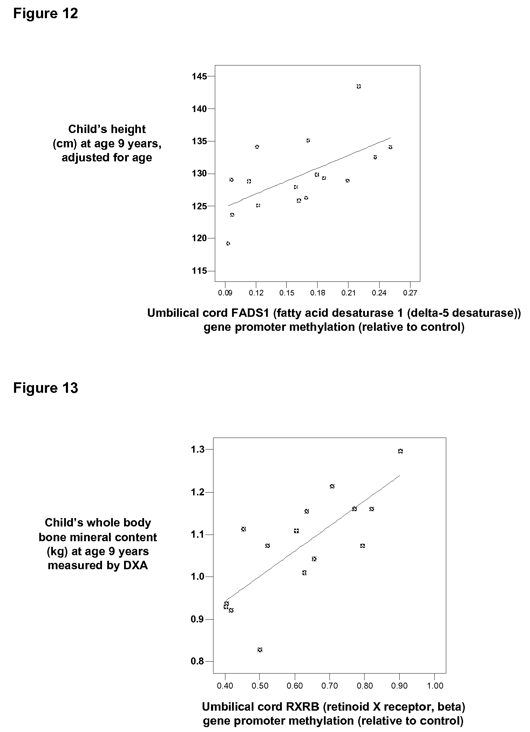

Figure 12 shows from the human studies described in Example 1, umbilical cord FADS1 (fatty acid desaturase 1 (delta-5 desaturase)) gene promoter methylation (relative to control) in relation to the child's height at age nine years. N=16; t=0.62, P=0.01 by Spearman correlation.
Figure 13 shows from the human studies described in Example 1, umbilical cord RXRB (retinoid X receptor, beta) gene promoter methylation (relative to control) in relation to the child's whole body bone mineral content at age nine years measured by dual energy X-ray absorptiometry (DXA). N=15; r=0.74, P=0.002 by Spearman correlation.

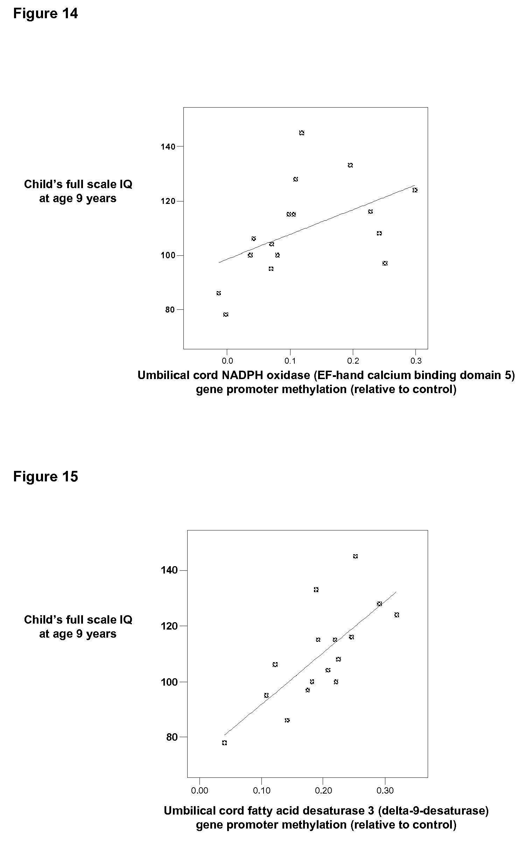

Figure 14 shows from the human studies described in Example 1, umbilical cord NOX5 (NADPH oxidase, EF-hand calcium binding domain 5) gene promoter methylation (relative to control) in relation to the child's full scale IQ at age nine years. N=16; r=0.64, P=0.008 by Spearman correlation.
Figure 15 shows from the human studies described in Example 1, umbilical cord FADS3 (fatty acid desaturase 3 (delta-9 desaturase)) gene promoter methylation (relative to control) in relation to the child's full scale IQ at age nine years. N=16; r=0.76, P=0.001 by Spearman correlation.

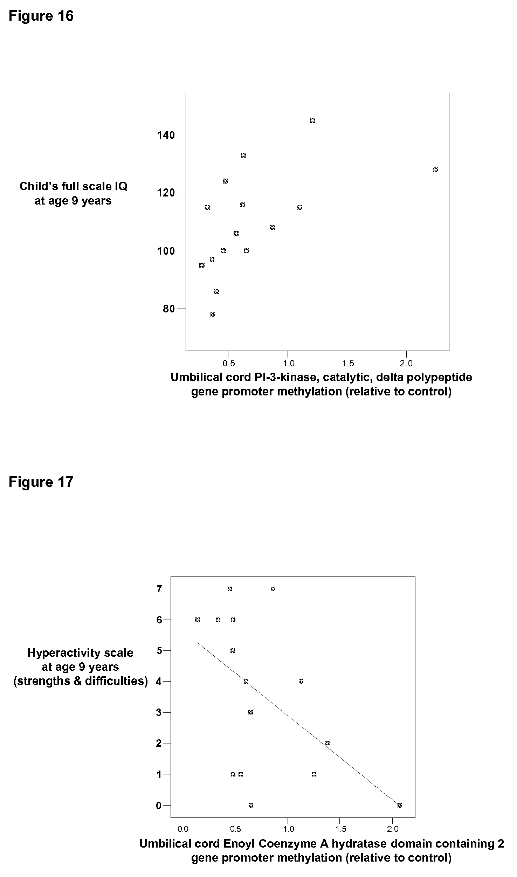

Figure 16 shows from the human studies described in Example 1, umbilical cord PIK3CD (PI-3-kinase, catalytic, delta polypeptide ) gene promoter methylation (relative to control) in relation to the child's full scale IQ at age nine years. N=15; i=0.66, P=0.007 by Spearman correlation.
Figure 17 shows from the human studies described in Example 1, umbilical cord ECHDC2 (Enoyl Coenzyme A hydratase domain containing 2) gene promoter methylation (relative to control) in relation to the child's score on the hyperactivity scale at age 9 years using the strengths and difficulties questionnaire. N=15; i=-0.72, P=0.005 by Spearman correlation.

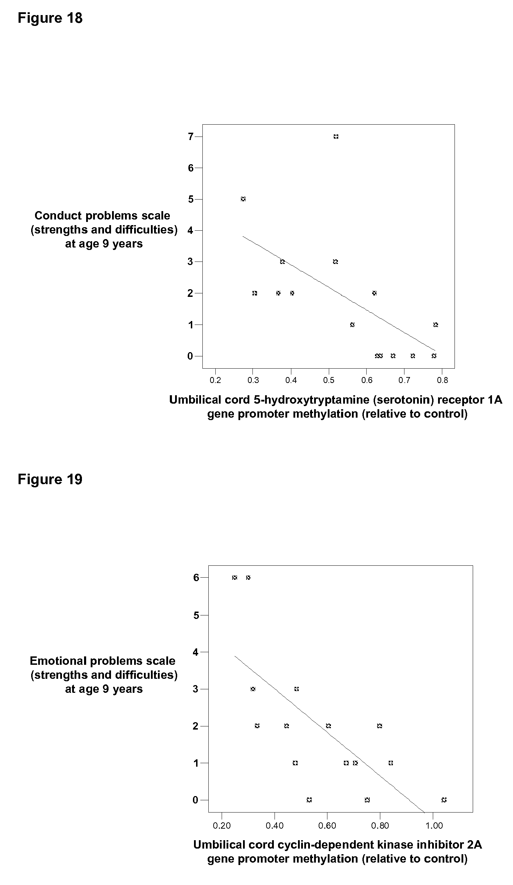

Figure 18 shows from the human studies described in Example 1, umbilical cord HTR1A (5-hydroxytryptamine (serotonin) receptor 1A) gene promoter methylation (relative to control) in relation to the child's score on the conduct problems scale at age 9 years using the strengths and difficulties questionnaire. N=15; r=-0.76, P=0.001 by Spearman correlation.
Figure 19 shows from the human studies described in Example 1, umbilical cord CDKN2A (cyclin-dependent kinase inhibitor 2A) gene promoter methylation (relative to control) in relation to the child's score on the emotional problems scale at age 9 years using the strengths and difficulties questionnaire. N=15; r=-0.74, P=0.002 by Spearman correlation.

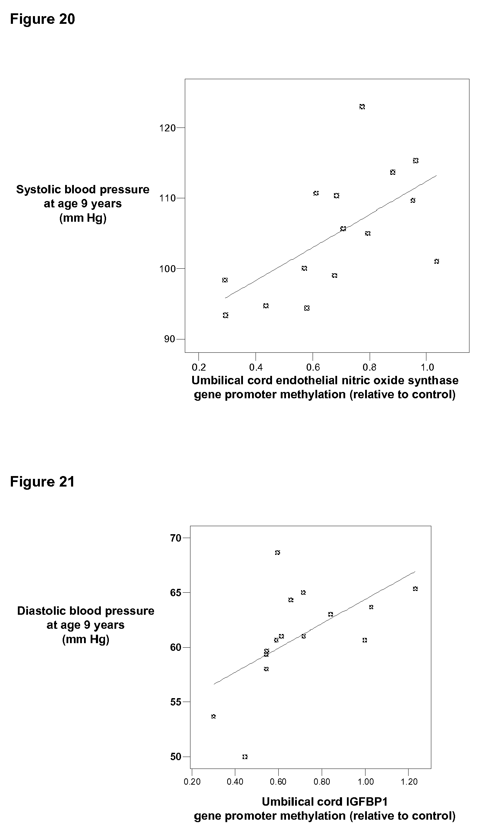

Figure 20 shows from the human studies described in Example 1, umbilical cord NOS3 (endothelial nitric oxide synthase) gene promoter methylation (relative to control) in relation to the child's systolic blood pressure at age nine years. N=15; r=0.68, P=0.005 by Spearman correlation.
Figure 21 shows from the human studies described in Example 1, umbilical cord IGFBP1 (insulin like growth factor binding protein-1) gene promoter methylation (relative to control) in relation to the child's diastolic blood pressure at age nine years. N=15; r=0.72, P=0.002 by Spearman correlation.

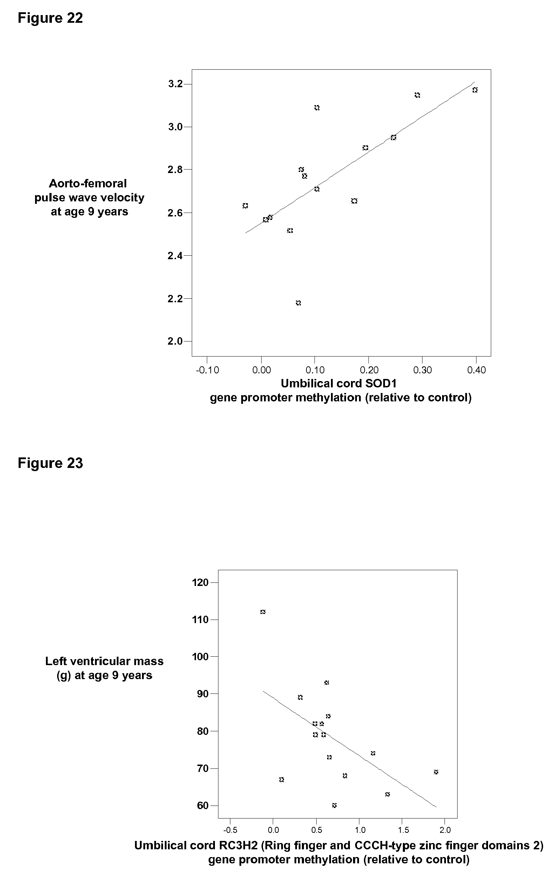

Figure 22 shows from the human studies described in Example 1, umbilical cord SOD1 (superoxide dismutase) gene promoter methylation (relative to control) in relation to the child's aorto-femoral pulse wave velocity at age nine years. N=14; r=0.82, P<0.001 by Spearman correlation.
Figure 23 shows from the human studies described in Example 1, umbilical cord RC3H2 (ring finger and CCCH-type zinc finger domains 2) gene promoter methylation (relative to control) in relation to the child's left ventricular mass at age nine years measured by echocardiography. N=15; r=-0.57, P=0.026 by Spearman correlation.

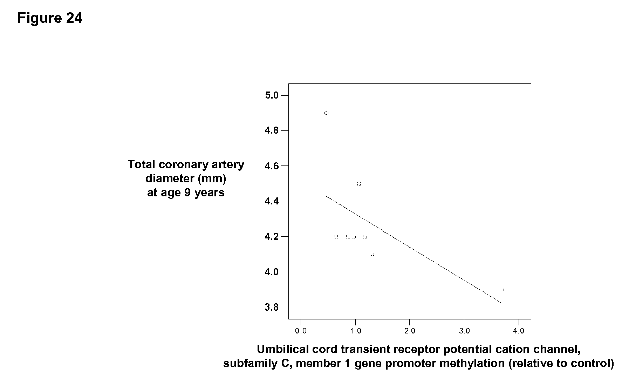

Figure 24 shows from the human studies described in Example 1, umbilical cord TRPC1 (transient receptor potential cation channel, subfamily C, member 1) gene promoter methylation (relative to control) in relation to the child's total coronary artery diameter at age nine years. N=8; r=-0.75, P=0.03 by Spearman correlation.

### Detailed Description

In general terms, this invention relates to a method of predicting, for individuals and populations of individuals, total body lean mass and/or percentage lean mass, or susceptibility for sarcopenia or impaired muscle function, and/or impaired skeletal development associated with reduced bone mineral content or a disease condition associated with low bone mineral content such as osteoporosis, using an epigenetic marker independent of changes in gene expression. Also disclosed herein is a method of predicting the propensity for individuals, and populations of individuals, to develop obesity or another phenotypic characteristic using an epigenetic marker independent of changes in gene expression. The ability to predict the likely occurrence of obesity or other disorders from an early age in individuals, coupled with targeted intervention, will provide a valuable pre-emptive means of controlling the effect of the phenotype on the health of the individual at a later age. Obesity increases the incidence of diabetes, heart disease etc, therefore a reduction in the likelihood that an individual will develop obesity in later life is beneficial not only for the individual concerned but also for the community as a whole.

From this perspective the disclosures herein can also be seen to provide methods for predicting the propensity of individuals, or populations of individuals, to develop phenotypes such as diabetes and heart disease for example.

Methods of the invention and as disclosed herein can also be applied to the agricultural and domestic animal sector. Early prediction of obesity, by way of example, in farm animals (cattle, sheep, pigs, chickens, deer) would enable farmers to select and / or manage animals so as to maximise production efficiency and thereby economic returns (e.g. carcass yield of lean meat and breed or slaughter decisions). Similarly early diagnosis of predisposition to obesity would enable interventions to manage the condition and maximise animal performance in the bloodstock industries (e.g. horses) and manage obesity in companion animals (e.g. cats and dogs).

As described in more detail in Reference Example 1, the inventors observed in rat adipose tissue that altered methylation of the glucocorticoid receptor gene, independent of any change in GR expression, provides at least a semi-permanent, if not permanent, marker that is predictive of obesity in later life. As noted above, altered methylation of the glucocorticoid receptor in the liver had previously been found to occur in neonates as a result of maternal undernutrition but was not known to be associated with obesity (Lillycrop et al. 2005, *ibid*.). The findings presented in Reference Example 1 were novel and unexpected as there is no genetic change/expression link between the methylation alteration and obesity. The alteration is observable in the adipose tissue of the individual and the at least semi-permanent nature of the alteration allowed the determination of the relationship between the methylation change and the phenotype to be determined by the inventors. It is anticipated that samples for such testing, whether in animals or humans, could be taken not only from adipose tissue but also, for example, from placenta, chorionic villus biopsy, umbilical cord, blood (including fetal and cord blood), hair follicles, buccal smears and muscle biopsies, or other such readily accessible tissues.

Moreover, further data as now presented in Example 1, links degree of methylation of additional genes with indicators of obesity in humans and illustrates that determination of such epigenetic change at birth or soon after can be used to predict propensity for obesity in later life. While such studies employed umbilical cord samples taken at birth and frozen, it is to be expected that alternative perinatal tissue samples may be employed which will provide genomic DNA of the individual of interest, e.g. blood taken shortly after birth or amniotic fluid.

While the above discussion has focused principally on obesity, it is emphasised that as further illustrated by Example 1 the concept of the inventors of using epigenetic change in genes in perinatal tissues as a marker for propensity for later development of particular phenotypic characteristics extends to a wide variety of characteristics as diverse as bone mineral content and density, neuro-behavioural characteristics and IQ. For further information on specific genes for which promoter methylation status in umbilical cord has been correlated with particular phenotypic characteristics at age 9, the reader is referred to the gene tables in Example 1. Those genes for which strong correlation is noted will be favoured for use in phenotype prediction, particularly in humans.

By using microarray analysis or other methods to identify methylated promoters, methylation of combinations of different promoters may be conveniently assessed enabling propensity for a single phenotypic characteristic to be determined or propensity for multiple phenotypic characteristics to be determined simultaneously using a single tissue sample, e.g. an umbilical cord sample. A commercially available microarray may be employed such as the NimbleGen Human ChiP Epigenetic Promoter Tiling Array. However, it may be preferred to use a microarray specifically designed to detect methylation of a combination of promoters associated with propensity for one or more phenotypic characteristics. Such phenotype-specific microarrays form a further disclosure. However, alternatively methylation-sensitive amplification, e.g. PCR amplification, may be carried out, particularly if the desire is to look at methylation status of a single gene or small number of genes strongly linked with one or more phenotypic characteristics. In this case, the gene or genes of interest will be amplified and the amplified nucleic acid treated with methylation-sensitive restriction enzymes, e.g. Aci1 and Hpa11, as described in more detail in ReferenceExample 1. A kit comprising primers and restriction enzymes suitable for carrying out methylation-sensitive amplification to detect methylation status of a combination of the promoter of the RXRA gene and the promoter of at least one other gene for predicting one or more of total body lean mass and/or percentage lean mass, or susceptibility for sarcopenia or impaired muscle function, and impaired skeletal development associated with reduced bone mineral content or a disease condition associated with low bone mineral content such as osteoporosis constitutes a still further aspect of the invention.

Once the propensity for exhibiting phenotypes has been determined in an individual, management of that individual's lifestyle (diet, behaviour, exercise, etc) can be undertaken to reduce the risk of the actual occurrence of any undesirable characteristics such as obesity or low mineral bone content associated with osteoporosis.

Where a method as disclosed herein identifies propensity for obesity in a newborn or child, then this may be used as a cue to also look at the nutritional status of the mother in view of the previously identified link between such propensity and poor maternal nutrition, and where poor maternal nutritional is found, improving the diet of the individual, e.g. by providing dietary advice and /or food supplements.

The methodology of the invention or as disclosed herein may be used to look at the occurrence of propensity for one or more phenotypic characteristics in a chosen population group and thereby used to identify external factors which contribute to the incidence of the characteristic(s), e.g. obesity, in the population of interest. In this way, information may be obtained useful in directing public health initiatives aimed at reducing the incidence of undesirable phenotypic characteristics in populations and thereby associated disorders such as diabetes, osteoporosis, sarcopenia, behavioural and mental disorders, hypertension, and cardiac problems.

The following examples illustrate the invention and the disclosures herein.

### Examples

### Reference Example 1

### Study design

A previously developed maternal undernutrition model of fetal programming was utilized in this study (Vickers et al, 2000, American Journal of Physiology 279:E83-E87). Virgin Wistar rats (age 100±5 days) were time mated using a rat estrous cycle monitor to assess the stage of estrous of the animals prior to introducing the male. After confirmation of mating, rats were housed individually in standard rat cages with free access to water. All rats were kept in the same room with a constant temperature maintained at 25°C and a 12-h light: 12-h darkness cycle. Animals were assigned to one of two nutritional groups: a) undernutrition (30% of ad-libitum) of a standard diet throughout gestation (UN group), b) standard diet ad-libitum throughout gestation (AD group). Food intake and maternal weights were recorded daily until the end of pregnancy. After birth, pups were weighed and litter size was adjusted to 8 pups per litter to assure adequate and standardized nutrition until weaning. Pups from undernourished mothers were cross-fostered on to dams that had received AD feeding throughout pregnancy. At weaning, AD and UN offspring were weight-matched and placed on either standard rat chow or a high fat diet (HF, Research Diets # 12451, 45%kcals as fat). At postnatal day 170, rats were fasted overnight and sacrificed by halothane anaesthesia followed by decapitation. Blood was collected into heparinised vacutainers and stored on ice until centrifugation and removal of supernatant for analysis. Tissues, including retroperitoneal fat, were dissected, weighed, immediately frozen in liquid nitrogen and store at -80 until analysis. All animal work was approved by the Animal Ethics Committee of the University of Auckland.

### Measurements

Body composition was assessed using dual energy x-ray absorbtiometry (DEXA, Hologic, Waltham, Massachusetts, USA).

### Analysis of mRNA expression

PPARα, PPARγ, AOX, CPT-1, lipoprotein lipase (LPL), leptin receptor (LR), glucocorticoid receptor and leptin mRNA concentrations were determined by RTPCR amplification and quantified by densitometry [Lillycrop et al. (2005)]. Briefly, total RNA was isolated from cells using TRIZOL reagent (InVitrogen), and 0.1 µg served as a template to prepare cDNA using 100 U Moloney-Murine Leukemia Virus reverse transcriptase. cDNA was amplified using primers specific to PPARα, PPARγ, AOX, CPT-1, LPL, LR, leptin and GR (Table 1). The PCR conditions in which the input cDNA was linearly proportional to the PCR product were initially established for each primer pair. One tenth of the cDNA sample was amplified for 25 cycles for the housekeeping gene ribosomal 18S and for 30 cycles for other genes. mRNA expression was normalized using the housekeeping gene ribosomal 18S RNA.

### Methylation-sensitive PCR.

The methylation status of genes was determined using methylation-sensitive PCR as described [Lillycrop et al. (2005)]. Briefly, genomic DNA (5 µg), isolated from adipose tissue using a QIAquick PCR Purification Kit (QIAGEN, Crawley, Sussex, UK) and treated with the methylation-sensitive restriction enzymes Aci1 and HpaII as instructed by the manufacturer (New England Biolabs, Hitchin, Hertfordshire, UK). The resulting DNA was then amplified using real-time PCR (Table 1), which was performed in a total volume of 25 µL with SYBR^{®} Green Jumpstart ready mix as described by the manufacturer (Sigma, Poole, Dorset, UK). As an internal control, the promoter region from the rat PPARγ2 gene, which contains no CpG islands and no Aci1 or HpaII recognition sites, was amplified. All CT values were normalized to the internal control.

Statistical analyses were carried out using SPSS (SPSS Inc., Chicago, USA) statistical packages. Differences between groups were determined by two-way factorial ANOVA (prenatal nutrition and postnatal diet as factors) followed by Bonferonni post-hoc analysis and data are shown as mean ± SEM.

### Results and Discussion

Referring to the Figures 1-5 (in which * is p<0.05, NS = not significant),
Figure 1 shows that the UNHF group has a significantly higher body weight gain on a high fat diet than control animals on the same diet. The increased body weight gain is indicative of diet-induced obesity as a result of developmental reprogramming of gene expression;
Figure 2 illustrates that total body mass fat as determined by DEXA analysis is significant in the UNHF group compared with control animals on a high fat diet. This is once again illustrative of diet-induced obesity as a result of developmental reprogramming;
Figure 3: Back fat is a readily dissectable depot of fat which is an *in situ* marker of total body adiposity. The back fat data shows a close relationship to whole body fat and further illustrates the increase in adiposity in UNHF animals compared with control animals on a high fat diet associated with developmental reprogramming:
Figure 4: The data illustrates that the post natal HF diet has no significant effect on GR promoter gene methylation status in progeny from rats that had access to feed ad libitum throughout pregnancy whereas in progeny fed the high fat diet from rats under nourished through pregnancy methylation was significantly reduced. More broadly the data show that a decrease in GR promoter methylation is directly associated with increased propensity to obesity; and
Figure 5: These data show that there was no significant effect of feeding a postnatal HF diet on GR expression with or without prenatal nutrient restriction. One explanation is that because gene expression requires the action of additional (transcription) factors, the offspring with lower GR methylation (UNHF) had potential for greater GR expression, but that in the absence of an appropriate stimulus GR expression was at a baseline levels.

### Example 1

### Samples

Umbilical cord samples were taken from pregnancies in the University of Southampton/UK Medical Research Council Princess Anne Hospital Nutrition Study.

### Methods - subjects and phenotyping

In 1991-2 Caucasian women aged >16 years with singleton pregnancies of <17 weeks' gestation were recruited at the Princess Anne Maternity Hospital in Southampton, UK; diabetics and those who had undergone hormonal treatment to conceive were excluded. In early (15 weeks of gestation) and late (32 weeks of gestation) pregnancy, we administered a dietary and lifestyle questionnaire to the women. Anthropometric data on the child were collected at birth and a 1-inch section of umbilical cord was collected and stored at -40°C. Gestational age was estimated from menstrual history and scan data. 559 children were followed-up at age nine months, when data on anthropometry and infant feeding were recorded. Collection and analysis of human umbilical cord samples was carried out with written informed consent from all subjects and under IRB approval from the Southampton and South West Hampshire Joint Research Ethics Committee.

When these children approached age nine years, we wrote to the parents of those still living in Southampton inviting the children to participate in a further study. Of 461 invited, 216 (47%) agreed to attend a clinic. Height was measured using a stadiometer and weight using digital scales (SECA Model No.835). The children underwent measurements of body composition by DXA (dual energy x-ray absorptiometry; Lunar DPX-L instrument using specific paediatric software; version 4.7c, GE Corporation, Madison, WI, USA). The instrument was calibrated every day and all scans were done with the children wearing light clothing. The short-term and long-term coefficients of variation of the instrument were 0.8% and 1.4% respectively. After a five-minute rest, systolic and diastolic blood pressures were measured three times on the left arm placed at the level of the heart whilst the child was seated. Measurements were made using a Dinamap 1846 (Critikon, UK), with manufacturer's recommended cuff sizes based on the child's mid upper arm circumference. The mean of the three measurements was used in the analysis.

Cognitive function was measured using the Wechsler Abbreviated Scale of Intelligence (WASI) (Wechsler D., Wechsler Abbreviated Scale of Intelligence. The Psychological Corporation, Sam Antonio, 1999) and psychological health was assessed with the Strengths and Difficulties Questionnaire (SDQ), which was completed by the mother (Goodman R. The Strengths and Diffculties Questionnaire: a research note. J. Child Psychol. Psychiatr. (1997) 38:581-586). The SDQ is made up of 5 subscales assessing prosocial behaviour, hyperactivity, emotional symptoms, conduct problems and peer problems.

Arterial compliance was measured by a non-invasive optical method that determines the transit time of the wave of dilatation propagating in the arterial wall, as a result of the pressure wave generated by contraction of the left ventricle. Measurement of the time taken for the wave to travel a known distance allows the velocity of the pulse wave to be calculated. The optical method has been validated against intra-arterial determinations of pressure wave velocity (1Bonner et al. Validation and use of an optical technique for the measurement of pulse wave velocity in conduit arteries. Fortschritt Berichte (1995) 107: 43-52). Pulse wave velocities were measured in two arterial segments, aorta to femoral, extending from the common carotid artery near the arch of the aorta into the femoral artery just below the inguinal ligament, and aorta to foot, extending to the posterior tibial artery. Pulse wave velocity is inversely related to the square root of the compliance of the vessel wall. High pulse wave velocity therefore indicates a stiffer arterial wall.

When pulse rate and blood pressure measurements indicated hemodynamic stability, transthoracic echocardiography (Acuson 128 XP and a 3.5MHz phased array transducer) was performed by a single ultrasonographer with the child in the left lateral recumbent position. Two dimensional, M-mode, and Doppler echocardiograms were recorded over five consecutive cardiac cycles and measurements were made off-line. Left ventricular mass (according to American Echocardiography Society convention) and total coronary artery diameter were measured as reported previously (Jiang B, Godfrey KM, Martyn CN, Gale CR (2006). Birth weight and cardiac structure in children. Pediatrics 117, e257-e261; Gale CR, Robinson SM, Harvey NC, Javaid MK, Jiang B, Martyn CN, Godfrey KM, Cooper C, Princess Anne Hospital Study Group. (2007) Maternal vitamin D status during pregnancy and child outcomes. Eur. J. Clin. Nutr. - in press).

### Methods - gene promoter methylation, assays in umbilical cord

Genomic DNA was extracted from the umbilical cord samples. We used an existing commercially available microarray (NimbleGen Human ChIP Epigenetic Promoter Tiling Array) to measure the degree of CpG methylation of putative promoters of 24,434 genes. This analyses 5-50 kb of promoter region for the comprehensive human genome in 2 arrays, tiling regions at 110 bp intervals and using variable length probes (~5/promoter). All annotated splice variants and alternative splice sites are represented on the NimbleGen array.

In consequence of the high cost of the arrays (£1000 per subject), our approach was to maximise insights from using 18 arrays while also obtaining essential information on reproducibility; we therefore undertook measurements on 16 subjects, randomly selected from 4 strata of IQ at age 9 years, one of whom had measurements performed in triplicate.

### Methods - initial data analysis

The intra-subject variability in median gene promoter methylation as compared with controls across ∼12,000 gene promoters for the subject with a triplet of repeat assays showed a standard deviation of 0.11, as compared with a between subjects standard deviation of 0.23. This suggests that measurement error is relatively small in comparison with the biological variability between subjects.

Our initial analyses focussed on 24 genes with established candidacy for cardiovascular and bone development (Appendix 1) and the 50 genes with greatest between subject variability in gene promoter methylation measured using the NimbleGen microarray (Appendix 2). Subsequent analyses examined 55 genes with established candidacy for neural, cellular and metabolic processes (Appendix 3). Using SPSS 12.0 for Windows we used non-parametric tests (Spearman rank correlation) to relate the degree of methylation of the gene promoters in umbilical cord to offspring phenotype at age 9 years.

### Summary of findings - body composition

### Childhood fat mass, proportionate fat mass and body fat distribution at age 9 years

For 27 of the 74 genes in our initial analyses and 32 of the 55 genes in subsequent analyses (see Table 1), we found evidence that the degree of gene promoter methylation was associated with the child's total and/or proportionate body fat mass and/or body fat distribution. The associations with total body fat mass were particularly strong for the NOS3 (endothelial nitric oxide synthase) gene (Spearman correlation coefficient r=0.66, P=0.007), the MMP2 (matrix metalloproteinase-2) gene (r=0.62, P=0.01) and the PI3KCD (phosphoinositide-3-kinase, catalytic, δ-polypeptide) gene (r=0.68, P=0.005; Figure 6). The associations with proportionate body fat mass were particularly strong for the MMP2 (matrix metalloproteinase-2) gene (r=0.66, P=0.005; Figure 7) and the HGSNAT (heparan-α-glucosaminide N-acetyltransferase) gene (r=0.73, P=0.002; Figure 8). The associations with body fat distribution were particularly strong for the DRD1IP (dopamine receptor D1 interacting protein) gene (Spearman correlation coefficient r=-0.55, P=0.026) and the SYN3 (synapsin III) gene (Spearman correlation coefficient r=-0.56, P=0.025). Linkage of such gene promoter methylation with indicators of obesity is expected to be of assistance in identifying individuals at risk of, for example, type-2 diabetes, metabolic syndrome, cardiovascular disease and other conditions for which obesity is recognised to be a risk factor.

**Table 1**

| | |
|---|---|
| AK090950 | KIAA1345 protein |
| C1orf185 | Chromosome 1 open reading frame 185 |
| CIB4 | Calcium and integrin binding family member 4 |
| ECHDC2 | Enoyl Coenzyme A hydratase domain containing 2 |
| EDN1 | Endothelin 1 |
| FLAD1 | FAD1 flavin adenine dinucleotide synthetase homolog |
| FLJ23577 | KPL2 protein |
| GLO1 | Glyoxalase I |
| HGSNAT | Heparan-alpha-glucosaminide N-acetyltransferase |
| HIVEP2 | HIV type I enhancer binding protein 2 |
| HSP90AB3P | Heat shock protein 90kDa alpha (cytosolic), class B member 3 (pseudogene) |
| IL8 | Interleukin-8 |
| ISG15 | ISG15 ubiquitin-like modifier |
| LOC401027 | Hypothetical protein LOC401027 |
| MCL1 | Myeloid cell leukemia sequence 1 (BCL2-related) |
| MMP2 | Matrix metalloproteinase-2 |
| NOS3 | Nitric oxide synthase 3 (endothelial cell) (eNOS) |
| NR3C1 | Glucocorticoid receptor; nuclear receptor subfamily 3, group C, member 1 |
| OGDH | Oxoglutarate (alpha-ketoglutarate) dehydrogenase (lipoamide) |
| OR5C1 | Olfactory receptor, family 5, subfamily C, member 1 |
| PI3KCD | Phosphoinositide-3-kinase, catalytic, delta polypeptide |
| PREB | Prolactin regulatory element binding |
| PSME4 | Proteasome (prosome, macropain) activator subunit 4 |
| RALGPS1 | Ral GEF with PH domain and SH3 binding motif 1 |
| TNF | Tumor necrosis factor (TNF superfamily, member 2)(TNFa) |
| TSN | Translin |
| VEGFA | Vascular endothelial growth factor A |
| CDK9 | cyclin-dependent kinase 9 (CDC2-related kinase) |
| CDKN1A | cyclin-dependent kinase inhibitor 1 A (p21, Cip1) |
| CDKN2A | cyclin-dependent kinase inhibitor 2A (melanoma, p16, inhibits CDK4) |
| CDKN2B | cyclin-dependent kinase inhibitor 2B (p15, inhibits CDK4) |
| CREB1 | cAMP responsive element binding protein 1 |
| DRD1IP | dopamine receptor D1 interacting protein |
| DRD2 | D(2) dopamine receptor |
| EGR1 | early growth response 1 |
| FADS1 | fatty acid desaturase 1 (delta-5 desaturase) |
| FADS2 | fatty acid desaturase 2 (delta-6-desaturase) |
| FADS3 | fatty acid desaturase 3 (delta-9-desaturase) |
| FAF1 | Fas (TNFRSF6) associated factor 1 |
| FLT1 | fms-related tyrosine kinase 1 (VEGF/vascular permeability factor receptor) |
| GABRB1 | gamma-aminobutyric acid (GABA) A receptor, beta 1 |
| GAD1 | glutamate decarboxylase 1 (brain, 67kDa) |
| GRIK2 | glutamate receptor, ionotropic, kainate 2 |
| GRIN3B | glutamate receptor, N-methyl-D-aspartate 3B (NMDA receptor subunit 3B) |
| HSFY1 | heat shock transcription factor, Y-linked 1 (more centromeric copy) |
| HSFY2 | HSFY2 heat shock transcription factor, Y linked 2 (more telomeric copy) |
| HTR2A | 5-hydroxytryptamine (serotonin) receptor 2A |
| IGFBP1 | insulin-like growth factor binding protein 1 |
| MAOA | monoamine oxidase A |
| MAPK1 | mitogen-activated protein kinase 1 (protein tyrosine kinase ERK2) |
| MYC | v-mvc myelocytomatosis viral oncogene homolog (avian) |
| RXRA | retinoid X receptor, alpha |
| RXRB | retinoid X receptor, beta |
| S100B | S100 calcium binding protein B |
| SCD | stearoyl-CoA desaturase (delta-9-desaturase) |
| SEMA4F | sema domain, Ig domain, TM domain & short cytoplasmic domain, (semaphorin) 4F |
| SLC6A4 | solute carrier family 6 (neurotransmitter transporter, serotonin), member 4 (5-HTT) |
| SYN3 | synapsin III |
| TNFRSF1A | tumor necrosis factor receptor superfamily, member 1A |

In mRNA gene expression studies, both the FADS1 and FADS2 genes have also been found to be upregulated in liver from neonatal rats of undernourished mothers.

### Childhood lean mass at age 9 years

For 34 of the 74 genes in our initial analyses and 29 of the 55 genes in subsequent analyses (see Table 2), we found evidence that the degree of gene promoter methylation was associated with child's total lean body mass and/or proportionate lean mass. Such gene methylation is of interest in relation to identifying individuals who may be susceptible to, for example sarcopenia and impaired muscle function. The associations with child's total lean body mass were particularly strong for the RC3H2 gene (ring finger and CCCH-type zinc finger domains 2) (r=-0.70, P=0.004; Figure 9), the EGR (early growth response 1) gene (r=0.66, P=0.008), the FTO (fat mass and obesity associated) gene (r=-0.53, P=0.034), the RXRB (retinoid X receptor, beta) gene (r=0.75, P=0.001) and the RXRA (retinoid X receptor, alpha) gene (r=0.76, P=0.001; Figure 10).

**Table 2**

| | |
|---|---|
| AK090950 | KIAA1345 protein |
| ATP2A3 | ATPase, Ca++ transporting, ubiquitous (=PMCA3) |
| BDP1 | B double prime 1, subunit of RNA polymerase III transcription initiation factor IIIB |
| C1orf185 | Chromosome 1 open reading frame 185 |
| CIB4 | Calcium and integrin binding family member 4 |
| COX6C | Cytochrome c oxidase subunit Vic |
| DEFB107A | Defensin, beta 107A |
| ECHDC2 | Enoyl Coenzyme A hydratase domain containing 2 |
| FCGR1B | Fc fragment of IgG, high affinity lb, receptor (CD64) |
| FLAD1 | FAD1 flavin adenine dinucleotide synthetase homolog |
| FLJ41821 | FLJ41821 protein |
| HGSNAT | Heparan-α-glucosaminide N-acetyltransferase |
| HIVEP2 | HIV type I enhancer binding protein 2 |
| HSD11 B2 | Hydroxysteroid (11-beta) dehydrogenase 2 |
| IL8 | Interleukin-8 |
| ISG15 | ISG15 ubiquitin-like modifier |
| KLHL5 | Kelch-like 5 (Drosophila) |
| LOC401027 | Hypothetical protein LOC401027 |
| MGC48628 | Similar to KIAA1680 protein (=hypothetical protein LOC401145) |
| MMP2 | Matrix metalloproteinase-2 |
| MYH11 | Smooth muscle myosin heavy chain 11 |
| NOS3 | Nitric oxide synthase 3 (endothelial cell) (eNOS) |
| NR3C1 | Glucocorticoid receptor; nuclear receptor subfamily 3, group C, member 1 |
| OGDH | Oxoglutarate (alpha-ketoglutarate) dehydrogenase (lipoamide) |
| OR5C1 | Olfactory receptor, family 5, subfamily C, member 1 |
| PIGK | Phosphatidylinositol glycan anchor biosynthesis, class K |
| PIK3CD | Phosphoinositide-3-kinase, catalytic, delta polypeptide |
| PREB | Prolactin regulatory element binding |
| RAI16 | Retinoic acid induced 16 |
| RALGPS1 | Ral GEF with PH domain and SH3 binding motif 1 |
| RC3H2 | Ring finger and CCCH-type zinc finger domains 2 |
| TSN | Translin |
| VCAM1 | Vascular cell adhesion molecule 1 |
| VEGFA | Vascular endothelial growth factor A |
| CDK9 | cyclin-dependent kinase 9 (CDC2-related kinase) |
| CDKN2A | cyclin-dependent kinase inhibitor 2A (melanoma, p16, inhibits CDK4) |
| CHRM1 | cholinergic receptor, muscarinic 1 |
| COMT | catechol-O-methyltransferase |
| DRD3 | dopamine receptor D3 |
| EGR1 | early growth response 1 |
| FADS1 | fatty acid desaturase 1 (delta-5 desaturase) |
| FADS2 | fatty acid desaturase 2 (delta-6-desaturase) |
| FADS3 | fatty acid desaturase 3 (delta-9-desaturase) |
| FAF1 | Fas (TNFRSF6) associated factor 1 |
| FLT1 | fms-related tyrosine kinase 1 (VEGF/vascular permeability factor receptor) |
| FOS | v-fos FBJ murine osteosarcoma viral oncogene homolog |
| FTO | fat mass and obesity associated |
| GABRB1 | gamma-aminobutyric acid (GABA) A receptor, beta 1 |
| GRIK2 | glutamate receptor, ionotropic, kainate 2 |
| GRIN3B | glutamate receptor, N-methyl-D-aspartate 3B (NMDA receptor subunit 3B) |
| HTR2A | 5-hydroxytryptamine (serotonin) receptor 2A |
| IGFBP1 | insulin-like growth factor binding protein 1 |
| MAOA | monoamine oxidase A |
| MAPK1 | mitogen-activated protein kinase 1 (protein tyrosine kinase ERK2) |
| MYC | v-myc myelocytomatosis viral oncogene homolog (avian) |
| OTX1 | orthodenticle homeobox 1 |
| RXRA | retinoid X receptor, alpha |
| RXRB | retinoid X receptor, beta |
| S100B | S100 calcium binding protein B |
| SCD | stearoyl-CoA desaturase (delta-9-desaturase) |
| SEMA4F | sema domain, Ig domain, TM domain & short cytoplasmic domain, (semaphorin) 4F |
| SLC6A3 | Sodium-dependent dopamine transporter (DA transporter) (DAT) |
| SLC6A4 | solute carrier family 6 (neurotransmitter transporter, serotonin), member 4 (5-HTT) |

### Childhood bore mineral content and height at age 9 years

We found evidence that gene promoter methylation of 35 genes was associated with the child's bone mineral content (see Table 3), including the ECHDC2 (Enoyl Coenzyme A hydratase domain containing 2) gene (r=0.65; P=0.015), the MAOA (monoamine oxidase A) gene (r=0.65, P=0.006), the RXRA (retinoid X receptor, alpha) gene (r=0.68, P=0.005) and the RXRB (retinoid X receptor, beta) gene (r=0.74, P=0.002; Figure 13). Hence, determination of methylation of these genes is of interest in predicting impaired skeletal development and propensity for disease states linked with low bone mineral content such as osteoporosis in later life. For 17 of the 74 genes in our initial analyses and 13 of the 55 genes in our subsequent analyses (see Table 4), we found evidence that the degree of gene promoter methylation was associated with child's height, including the ESR1 (estrogen receptor-α) gene (r=0.51, P=0.05; Figure 11), the FADS1 (fatty acid desaturase 1 (delta-5 desaturase)) gene (r=0.62, P=0.01; Figure 12) and the OGDH (oxoglutarate dehydrogenase) gene (r=0.53, P=0.04). Determining methylation of such promotors may thus be utilised in identifying individuals liable to have impaired linear growth and may be of particular benefit at a very early age.

**Table 3**

| | |
|---|---|
| DEFB107A | Defensin, beta 107A |
| ECHDC2 | Enoyl Coenzyme A hydratase domain containing 2 |
| FLAD1 | FAD1 flavin adenine dinucleotide synthetase homolog |
| FLJ23577 | KPL2 protein |
| HGSNAT | Heparan-alpha-glucosaminide N-acetyltransferase |
| LOC646870 | Hypothetical protein LOC646870 |
| MCL1 | Myeloid cell leukemia sequence 1 (BCL2-related) |
| MMP9 | Matrix metallopeptidase 9 (gelatinase B, 92kDa gelatinase, 92kDa type IV collagenase) |
| NOS3 | Nitric oxide synthase 3 (endothelial cell) (eNOS) |
| PI3KCD | Phosphoinositide-3-kinase, catalytic, delta polypeptide |
| PPARG | Peroxisome proliferator-activated receptor gamma |
| PREB | Prolactin regulatory element binding |
| RALGPS1 | Ral GEF with PH domain and SH3 binding motif 1 |
| TNF | Tumor necrosis factor (TNF superfamily, member 2)(TNFa) |
| TSN | Translin |
| VEGFA | Vascular endothelial growth factor A |
| CDK9 | cyclin-dependent kinase 9 (CDC2-related kinase) |
| CDKN1A | cyclin-dependent kinase inhibitor 1 A (p21, Cip1) |
| CDKN2A | cyclin-dependent kinase inhibitor 2A (melanoma, p16, inhibits CDK4) |
| CDKN2B | cyclin-dependent kinase inhibitor 2B (p15, inhibits CDK4) |
| CDKN2C | cyclin-dependent kinase inhibitor 2C (p18, inhibits CDK4) |
| EGR1 | early growth response 1 |
| FADS3 | fatty acid desaturase 3 (delta-9-desaturase) |
| FAF1 | Fas (TNFRSF6) associated factor 1 |
| FLT1 | fms-related tyrosine kinase 1 (VEGF/vascular permeability factor receptor) |
| GAD1 | glutamate decarboxylase 1 (brain, 67kDa) |
| GRIK2 | glutamate receptor, ionotropic, kainate 2 |
| IGFBP1 | insulin-like growth factor binding protein 1 |
| MAOA | monoamine oxidase A |
| MYC | v-myc myelocytomatosis viral oncogene homolog (avian) |
| RXRA | retinoid X receptor, alpha |
| RXRB | retinoid X receptor, beta |
| SCD | stearoyl-CoA desaturase (delta-9-desaturase) |
| SEMA4F | sema domain, Ig domain, TM domain & short cytoplasmic domain, (semaphorin) 4F |
| SLC6A4 | solute carrier family 6 (neurotransmitter transporter, serotonin), member 4 (5-HTT) |

**Table 4**

| | |
|---|---|
| AK090950 | KIAA1345 protein |
| ECHDC2 | Enoyl Coenzyme A hydratase domain containing 2 |
| ESR1 | Estrogen receptor 1 (alpha) |
| FLAD1 | FAD1 flavin adenine dinucleotide synthetase homolog |
| FLJ23577 | KPL2 protein |
| HSP90AB3P | Heat shock protein 90kDa alpha (cytosolic), class B member 3 (pseudogene) |
| IL8 | Interleukin 8 |
| ISG15 | ISG15 ubiquitin-like modifier |
| KLHL5 | Kelch-like 5 (Drosophila) |
| NOS3 | Nitric oxide synthase 3 (endothelial cell) (eNOS) |
| OGDH | Oxoglutarate (alpha-ketoalutarate) dehydroyenase (lipoamide) |
| OR2G2 | Olfactory receptor, family 2, subfamily G, member 2 |
| PCDH1 | Protocadherin 1 (cadherin-like 1) |
| PI3KCD | Phosphoinositide-3-kinase, catalytic, delta polypeptide |
| RALGPS1 | Ral GEF with PH domain and SH3 binding motif 1 |
| TPM3 | Tropomyosin 3 |
| TSN | Translin |
| CDK9 | cyclin-dependent kinase 9 (CDC2-related kinase) |
| CDKN2C | cyclin-dependent kinase inhibitor 2C (p18, inhibits CDK4) |
| EGR1 | early growth response 1 |
| FADS1 | fatty acid desaturase 1 (delta-5 desaturase) |
| FAF1 | Fas (TNFRSF6) associated factor 1 |
| HTR1A | 5-hydroxytryptamine (serotonin) receptor 1A |
| MAOA | monoamine oxidase A |
| MYC | v-myc myelocytomatosis viral oncogene homolog (avian) |
| RXRA | retinoid X receptor, alpha |
| RXRB | retinoid X receptor, beta |
| SEMA4F | sema domain, Ig domain, TM domain & short cytoplasmic domain, (semaphorin) 4F |
| SYN1 | synapsin I |
| SYN2 | synapsin II |

### Summary of findings - cognitive and neuro-behavioural status

### Childhood IQ at age 9 years

For 20 of the 74 genes in our initial analyses and 25 of the 55 genes in our subsequent analyses (see Table 5), we found evidence that the degree of gene promoter methylation was associated with child's IQ. The associations with full scale IQ were particularly strong for the NOX5 (NADPH oxidase, EF-hand calcium binding domain 5) gene (r=0.64, P=0.008; Figure 14), the CDKN2A (cyclin-dependent kinase inhibitor 2A) gene (r=0.68, P=0.005), the FADS3 (fatty acid desaturase 3 (delta-9 desaturase)) gene (r=0.76, P=0.001; Figure 15) and for the PI3KCD (phosphoinositide-3-kinase, catalytic, δ-polypeptide) gene (r=0.66, P=0.007; Figure 16).

**Table 5**

| | |
|---|---|
| ATP2A3 | ATPase, Ca++ transporting, ubiquitous (=PMCA3) |
| ATP2B1 | ATPase, Ca++ transporting, plasma membrane 1 (=PMCA1) |
| C1orf185 | Chromosome 1 open reading frame 185 |
| ECHDC2 | Enoyl Coenzyme A hydratase domain containing 2 |
| HEMGN | Hemogen |
| HGSNAT | Heparan-alpha-glucosaminide N-acetyltransferase |
| HSD11B2 | Hydroxysteroid (11-beta) dehydrogenase 2 |
| IGF1R | Insulin-like growth factor 1 receptor |
| IL8 | Interleukin 8 |
| ISG15 | ISG15 ubiquitin-like modifier |
| MCL1 | Myeloid cell leukemia sequence 1 (BCL2-related) |
| MMP2 | Matrix metallopeptidase 2 (gelatinase A, 72kDa gelatinase, 72kDa type IV collagenase) |
| NOS3 | Nitric oxide synthase 3 (endothelial cell) (eNOS) |
| NOX5 | NADPH oxidase, EF-hand calcium binding domain 5 |
| PI3KCD | Phosphoinositide-3-kinase, catalytic, delta polypeptide |
| OGDH | Oxoglutarate (alpha-ketoglutarate) dehydrogenase (lipoamide) |
| PPARG | Peroxisome proliferator-activated receptor gamma |
| QSCN6 | Quiescin Q6 |
| RAI16 | Retinoic acid induced 16 |
| VEGFA | Vascular endothelial growth factor A |
| CDK4 | cyclin-dependent kinase 4 |
| CDKN2A | cyclin-dependent kinase inhibitor 2A (melanoma, p16, inhibits CDK4) |
| CHRM1 | cholinergic receptor, muscarinic 1 |
| COMT | catechol-O-methyltransferase |
| DRD2 | D(2) dopamine receptor |
| EGR1 | early growth response 1 |
| FADS1 | fatty acid desaturase 1 (delta-5 desaturase) |
| FADS2 | fatty acid desaturase 2 (delta-6-desaturase) |
| FADS3 | fatty acid desaturase 3 (delta-9-desaturase) |
| GABRB1 | gamma-aminobutyric acid (GABA) A receptor, beta 1 |
| GRIN3B | glutamate receptor, N-methyl-D-aspartate 3B (NMDA receptor subunit 3B) |
| HTR1A | 5-hydroxytryptamine (serotonin) receptor 1A |
| HTR2A | 5-hydroxytryptamine (serotonin) receptor 2A |
| MAOA | monoamine oxidase A |
| MAPK1 | mitogen-activated protein kinase 1 (protein tyrosine kinase ERK2) |
| MYC | v-myc myelocytomatosis viral oncogene homolog (avian) |
| OTX1 | orthodenticle homeobox 1 |
| PGF | placental growth factor, vascular endothelial growth factor-related protein |
| RELN | reelin |
| RXRA | retinoid X receptor, alpha |
| SLC6A3 | Sodium-dependent dopamine transporter (DA transporter) (DAT) |
| SLC6A4 | solute carrier family 6 (neurotransmitter transporter, serotonin), member 4 (5-HTT) |
| SYN1 | synapsin I |
| SYN3 | synapsin III |
| TNFRSF1A | tumor necrosis factor receptor superfamily, member 1A |

### Childhood neuro-behavioural status at age 9 years

Results also indicate that degree of gene promoter methylation can be used as a useful marker for neuro-behavioural disorders including, but not limited to hyperactivity, emotional problems, conduct problems, peer problems and/or total difficulties. For 48 of the 74 genes in our initial analyses and 34 of the 55 genes in our subsequent analyses (See Table 6), we found evidence that the degree of gene promoter methylation was associated with child's score on hyperactivity, emotional problems, conduct problems, peer problems and/or total difficulties scales. The associations with hyperactivity score were particularly strong for the IL8 (interleukin-8) gene (r=-0.77, P=0.002) and the ECHDC2 (Enoyl Coenzyme A hydratase domain containing 2) gene (r=-0.72, P=0.005; Figure 17). The associations with the conduct problem scores were particularly strong for the CDKN2C (cyclin-dependent kinase inhibitor 2C) gene (r=-0.67, P=0.007), the CHRM (cholinergic receptor, muscarinic 1) gene (r=-0.65, P=0.007) and the HTR1A (5-hydroxytryptamine (serotonin) receptor 1A) gene (r=-0.76, P=0.001; Figure 18). Association with the emotional problems score was particularly strong for the CDKN2A (cyclin-dependent kinase inhibitor 2A) gene (r=-0.74, P=0.002; Figure 19).

**Table 6**

| | |
|---|---|
| ACTC1 | Cardiac muscle alpha actin 1 |
| AK090950 | KIAA1345 protein |
| AK128539 | Homo sapiens cDNA FLJ46698 fis, clone TRACH3013684 |
| ATP2A3 | ATPase, Ca++ transporting, ubiquitous (=PMCA3) |
| ATP2B1 | ATPase, Ca++ transporting, plasma membrane 1 (=PMCA1) |
| CIB4 | Calcium and integrin binding family member 4 |
| C1 orf185 | Chromosome 1 open reading frame 185 |
| DEFB107A | Defensin, beta 107A |
| ECHDC2 | Enoyl Coenzyme A hydratase domain containing 2 |
| ESR1 | Estrogen receptor 1 (alpha) |
| FLAD1 | FAD1 flavin adenine dinucleotide synthetase homolog |
| FLJ13231 | Hypothetical protein FLJ13231 |
| FLJ23577 | KPL2 protein |
| GLO1 | Glyoxalase I |
| HGSNAT | Heparan-alpha-glucosaminide N-acetyltransferase |
| HIVEP2 | HIV type I enhancer binding protein 2 |
| HSD11 B2 | Hydroxysteroid (11-beta) dehydrogenase 2 |
| HSP90AB3P | Heat shock protein 90kDa alpha (cytosolic), class B member 3 (pseudogene) |
| hTAK1 | Human nuclear receptor hTAK1 |
| IL8 | Interleukin 8 |
| ISG15 | ISG15 ubiquitin-like modifier |
| KLHL5 | Kelch-like 5 (Drosophila) |
| LOC401027 | Hypothetical protein LOC401027 |
| LOC644936 | Similar to cytoplasmic beta-actin |
| LOC645156 | Hypothetical protein LOC645156 |
| MCL1 | Myeloid cell leukemia sequence 1 (BCL2-related) |
| MGC48628 | Similar to KIAA1680 protein (=hypothetical protein LOC401145) |
| MMP2 | Matrix metallopeptidase 2 (gelatinase A, 72kDa gelatinase, 72kDa type IV collagenase) |
| MMP9 | Matrix metallopeptidase 9 (gelatinase B, 92kDa gelatinase, 92kDa type IV collagenase) |
| MYOCD | Myocardin |
| NDUFA5 | NADH dehydroyenase (ubiquinone) 1 alpha subcomplex, 5, 13kDa |
| NOS3 | Nitric oxide svnthase 3 (endothelial cell) (eNOS) |
| NR3C1 | Glucocorticoid receptor; nuclear receptor subfamily 3, group C, member 1 |
| OGDH | Oxoglutarate (alpha-ketoglutarate) dehydrogenase (lipoamide) |
| OR2G2 | Olfactory receptor, family 2, subfamily G, member 2 |
| OR5C1 | Olfactory receptor, family 5, subfamily C, member 1 |
| PCDH1 | Protocadherin 1 (cadherin-like 1) |
| PIK3CD | Phosphoinositide-3-kinase, catalytic, delta polypeptide |
| PSME4 | Proteasome (prosome, macropain) activator subunit 4 |
| PREB | Prolactin regulatory element binding |
| QSCN6 | Quiescin Q6 |
| RALGPS1 | Ral GEF with PH domain and SH3 binding motif 1 |
| SOD1 | Superoxide dismutase 1, soluble (amyotrophic lateral sclerosis 1 (adult)) |
| TNF | Tumor necrosis factor (TNF superfamily, member 2)(TNFa) |
| TPM3 | Tropomyosin 3 |
| TSN | Translin |
| VEGFA | Vascular endothelial growth factor A |
| VCAM1 | Vascular cell adhesion molecule 1 |
| CDK9 | cyclin-dependent kinase 9 (CDC2-related kinase) |
| CDKN1A | cyclin-dependent kinase inhibitor 1 A (p21, Cip1) |
| CDKN2A | cyclin-dependent kinase inhibitor 2A (melanoma, p16, inhibits CDK4) |
| CDKN2B | cyclin-dependent kinase inhibitor 2B (p15, inhibits CDK4) |
| CDKN2C | cyclin-dependent kinase inhibitor 2C (p18, inhibits CDK4) |
| CHRM1 | cholinergic receptor, muscarinic 1 |
| COMT | catechol-O-methyltransferase |
| CREB1 | cAMP responsive element binding protein 1 |
| DRD1IP | dopamine receptor D1 interacting protein |
| DRD2 | D(2) dopamine receptor |
| EGR1 | early growth response 1 |
| FADS1 | fatty acid desaturase 1 (delta-5 desaturase) |
| FADS2 | fatty acid desaturase 2 (delta-6-desaturase) |
| FADS3 | fatty acid desaturase 3 (delta-9-desaturase) |
| FAF1 | Fas (TNFRSF6) associated factor 1 |
| GRIK2 | glutamate receptor, ionotropic, kainate 2 |
| HTR1A | 5-hydroxytryptamine (serotonin) receptor 1A |
| HTR4 | 5-hydroxytryptamine (serotonin) receptor 4 |
| IGFBP1 | insulin-like growth factor binding protein 1 |
| MAOA | monoamine oxidase A |
| MYC | v-myc myelocytomatosis viral oncogene homolog (avian) |
| NTRK2 | neurotrophic tyrosine kinase, receptor, type 2 |
| OTX1 | orthodenticle homeobox 1 |
| PGF | placental growth factor, vascular endothelial growth factor-related protein |
| RELN | reelin |
| RXRA | retinoid X receptor, alpha |
| RXRG | retinoid X receptor, gamma |
| S100B | S100 calcium binding protein B |
| SEMA4F | sema domain, Ig domain, TM domain & short cytoplasmic domain, (semaphorin) 4F |
| SLC6A3 | Sodium-dependent dopamine transporter (DA transporter) (DAT) |
| SYN1 | synapsin I |
| SYN2 | synapsin II |
| SYN3 | synapsin III |
| TNFRSF1A | tumor necrosis factor receptor superfamily, member 1A |

### Summary of findings - cardiovascular structure and function

### Childhood blood pressure at age 9 years

For 31 of the 74 genes in our initial analyses and 27 of the 55 genes in our subsequent analyses (see Table 7), we found evidence that the degree of gene promoter methylation was associated with child's systolic and/or diastolic blood pressure. Methylation of such gene promoters is therefore of interest in predicting propensity for hypertension. The associations with child's systolic blood pressure were particularly strong for the NOS3 (endothelial nitric oxide synthase) gene (r=0.68, P=0.005; see Figure 20), the NR3C1 (glucocorticoid receptor; nuclear receptor subfamily 3, group C, member 1) gene (i=0.62, P=0.01), the FADS3 (fatty acid desaturase-3) gene (r=0.64, P=0.007), the SEMA4F (sema domain, Ig domain, TM domain and short cytoplasmic domain, (semaphorin) 4F) gene (i=0.65, P=0.009) and the IL8 (interleukin-8) gene (r=0.71, P=0.003). The association with child's diastolic blood pressure was particularly strong for the IGFBP1 (insulin like growth factor binding protein-1) gene (i=0.72, P=0.002; Figure 21).

**Table 7**

| | |
|---|---|
| AK090950 | KIAA1345 protein |
| ANXA4 | Annexin A4 |
| CIB4 | Calcium and integrin binding family member 4 |
| DEFB107A | Defensin, beta 107A |
| ECHDC2 | Enoyl Coenzyme A hydratase domain containing 2 |
| EDN1 | Endothelin 1 |
| ESR1 | Estrogen receptor 1 (alpha) |
| FLAD1 | FAD1 flavin adenine dinucleotide synthetase homolog |
| GLO1 | Glyoxalase I |
| HGSNAT | Heparan-alpha-glucosaminide N-acetyltransferase |
| IGF1R | Insulin-like growth factor 1 receptor |
| IL8 | Interleukin 8 |
| KLHL5 | Kelch-like 5 (Drosophila) |
| LOC401027 | Hypothetical protein LOC401027 |
| MCL1 | Myeloid cell leukemia sequence 1 (BCL2-related) |
| MGC48628 | Similar to KIAA1680 protein (=hypothetical protein LOC401145) |
| MMP9 | Matrix metallopeptidase 9 (gelatinase B, 92kDa gelatinase, 92kDa type IV collaqenase) |
| MYH11 | Smooth muscle myosin heavy chain 11 |
| NDUFA5 | NADH dehydrogenase (ubiquinone) 1 alpha subcomplex, 5, 13kDa |
| NOS3 | Nitric oxide synthase 3 (endothelial cell) (eNOS) |
| NR3C1 | Glucocorticoid receptor; nuclear receptor subfamily 3, group C, member 1 |
| OGDH | Oxoglutarate (alpha-ketoglutarate) dehydrogenase (lipoamide) |
| OR2G2 | Olfactory receptor, family 2, subfamily G, member 2 |
| PIK3CD | Phosphoinositide-3-kinase, catalytic, delta polypeptide |
| PPARG | Peroxisome proliferator-activated receptor gamma |
| PREB | Prolactin regulatory element binding |
| QSCN6 | Quiescin Q6 |
| RTN4 | Reticulon 4 |
| TRPC1 | Transient receptor potential cation channel, subfamily C, member 1 |
| TSN | Translin |
| VEGFA | Vascular endothelial growth factor A |
| CDK10 | cyclin-dependent kinase (CDC2-like) 10 |
| CDK9 | cyclin-dependent kinase 9 (CDC2-related kinase) |
| CDKN1A | cyclin-dependent kinase inhibitor 1 A (p21, Cip1) |
| CDKN2A | cyclin-dependent kinase inhibitor 2A (melanoma, p16, inhibits CDK4) |
| COMT | catechol-O-methyltransferase |
| CREB1 | cAMP responsive element binding protein 1 |
| EGR1 | early growth response 1 |
| FADS1 | fatty acid desaturase 1 (delta-5 desaturase) |
| FADS2 | fatty acid desaturase 2 (delta-6-desaturase) |
| FADS3 | fatty acid desaturase 3 (delta-9-desaturase) |
| FAF1 | Fas (TNFRSF6) associated factor 1 |
| FLT1 | fms-related tyrosine kinase 1 (VEGF/vascular permeability factor receptor) |
| GABRB3 | gamma-aminobutyric acid (GABA) A receptor, beta 3 |
| HSFY2 | HSFY2 heat shock transcription factor, Y linked 2 (more telomeric copy) |
| HTR2A | 5-hydroxytryptamine (serotonin) receptor 2A |
| IGFBP1 | insulin-like growth factor binding protein 1 |
| MYC | v-myc myelocytomatosis viral oncogene homolog (avian) |
| OTX1 | orthodenticle homeobox 1 |
| PGF | placental growth factor, vascular endothelial growth factor-related protein |
| PPARD | peroxisome proliferator-activated receptor delta |
| RXRA | retinoid X receptor, alpha |
| RXRB | retinoid X receptor, beta |
| SEMA4F | sema domain, Ig domain, TM domain & short cytoplasmic domain, (semaphorin) 4F |
| SLC6A3 | Sodium-dependent dopamine transporter (DA transporter) (DAT) |
| SLC6A4 | solute carrier family 6 (neurotransmitter transporter, serotonin), member 4 (5-HTT) |
| SYN1 | synapsin I |
| TNFRSF1A | tumor necrosis factor receptor superfamily, member 1A |

### Childhood aorto-femoral pulse wave velocity and carotid artery intima media thickness at age 9 years

For 25 of the 74 genes in our initial analyses and 22 of the 55 genes in our subsequent analyses (see Table 8), we found evidence that the degree of gene promoter methylation was associated with child's aorto-femoral pulse wave velocity and/or carotid artery intima media thickness. Identification of such association may enable intervention to reduce disease risk associated with circulation problems, particularly atherosclerosis. The associations with child's aorto-femoral pulse wave velocity were particularly strong for the ATP2B1 (ATPase, Ca++ transporting, plasma membrane 1 (PMCA1)) gene (r=0.68, P=0.005) and the SOD1 (superoxide dismutase) gene (r=0.82, P<0.001, see Figure 22). Association with carotid artery intima media thickness was particularly strong for the CREB1 (cAMP responsive element binding protein 1) gene (r=-0.58, P=0.024).

**Table 8**

| | |
|---|---|
| ATP2B1 | ATPase, Ca++ transporting, plasma membrane 1 (=PMCA1) |
| ECHDC2 | Enoyl Coenzyme A hydratase domain containing 2 |
| EDN1 | Endothelin 1 |
| ESR1 | Estrogen receptor 1 (alpha) |
| FCGR1B | Fc fragment of IgG, high affinity Ib, receptor (CD64) |
| FLAD1 | FAD1 flavin adenine dinucleotide synthetase homolog |
| FLJ41821 | FLJ41821 protein |
| HEMGN | Hemogen |
| KLHL5 | Kelch-like 5 (Drosophila) |
| LOC644936 | Similar to cytoplasmic beta-actin |
| LOC646870 | Hypothetical protein LOC646870 |
| MGC48628 | Similar to KIAA1680 protein (=hypothetical protein LOC401145) |
| MMP2 | Matrix metallopeptidase 2 (gelatinase A, 72kDa gelatinase, 72kDa type IV collaqenase) |
| MYH11 | Smooth muscle myosin heavy chain 11 |
| MYOCD | Myocardin |
| NDUFA5 | NADH dehydrogenase (ubiquinone) 1 alpha subcomplex, 5, 13kDa |
| OGDH | Oxoglutarate (alpha-ketoglutarate) dehydrogenase (lipoamide) |
| POLQ | Polymerase (DNA directed), theta |
| PPARG | Peroxisome proliferator-activated receptor gamma |
| QSCN6 | Quiescin Q6 |
| RAI16 | Retinoic acid induced 16 |
| RALGPS1 | Ral GEF with PH domain and SH3 binding motif 1 |
| RTN4 | Reticulon 4 |
| SOD1 | Superoxide dismutase 1, soluble (amyotrophic lateral sclerosis 1 (adult)) |
| VEGFA | Vascular endothelial growth factor A |
| CDK4 | cyclin-dependent kinase 4 |
| CDKN2C | cyclin-dependent kinase inhibitor 2C (p18, inhibits CDK4) |
| CHRM1 | cholinergic receptor, muscarinic 1 |
| COMT | catechol-O-methyltransferase |
| CREB1 | cAMP responsive element binding protein 1 |
| DRD1IP | dopamine receptor D1 interacting protein |
| DRD2 | D(2) dopamine receptor |
| FADS1 | fatty acid desaturase 1 (delta-5 desaturase) |
| FADS2 | fatty acid desaturase 2 (delta-6-desaturase) |
| FAF1 | Fas (TNFRSF6) associated factor 1 |
| HSFY1 | heat shock transcription factor, Y-linked 1 (more centromeric copy) |
| HSFY2 | HSFY2 heat shock transcription factor, Y linked 2 (more telomeric copy) |
| IGFBP1 | insulin-like growth factor binding protein 1 |
| MAPK1 | mitogen-activated protein kinase 1 (protein tyrosine kinase ERK2) |
| NTRK2 | neurotrophic tyrosine kinase, receptor, type 2 |
| RELN | reelin |
| RXRG | retinoid X receptor, gamma |
| SEMA4F | sema domain, Ig domain, TM domain & short cytoplasmic domain, (semaphorin) 4F |
| SLC6A3 | Sodium-dependent dopamine transporter (DA transporter) (DAT) |
| SLC6A4 | solute carrier family 6 (neurotransmitter transporter, serotonin), member 4 (5-HTT) |
| SYN1 | synapsin I |
| TNFRSF1A | tumor necrosis factor receptor superfamily, member 1A |

### Childhood left ventricular mass at age 9 years

For 7 of the 74 genes in our initial analyses and 3 of the 55 genes in our subsequent analyses (see Table 9), we found evidence that the degree of gene promoter methylation was associated with child's left ventricular mass. Determination of methylation of such genes is therefore of interest in predicting propensity for left ventricular hypertrophy. The associations were particularly strong for the KLHL5 (Kelch-like 5) gene (r=-0.67, P=0.006) and the RC3H2 (Ring finger and CCCH-type zinc finger domains 2) gene (i=-0.57, P=0.026; see Figure 23 ).

**Table 9**

| | |
|---|---|
| C1orf185 | Chromosome 1 open reading frame 185 |
| ISG15 | ISG15 ubiquitin-like modifier |
| KLHL5 | Kelch-like 5 (Drosophila) |
| MGC12538 | Hypothetical protein MGC12538 |
| PCDH1 | Protocadherin 1 (cadherin-like 1) |
| POLQ | Polymerase (DNA directed), theta |
| RC3H2 | Ring finger and CCCH-type zinc finger domains 2 |
| GAD1 | glutamate decarboxylase 1 (brain, 67kDa) |
| MAPK1 | mitogen-activated protein kinase 1 (protein tyrosine kinase ERK2) |
| RXRB | retinoid X receptor, beta |

### Childhood coronary artery diameter at age 9 years

For 13 of the 74 genes in our initial analyses and 14 of the 55 genes in our subsequent analyses (see Table 10), we found evidence that the degree of gene promoter methylation was associated with child's total coronary *artery* diameter. Early age identification of propensity for small coronary *artery* clearly has important implications for combating coronary heart disease. The associations were particularly strong for the TRPC1 (Transient receptor potential cation channel, subfamily C, member 1) gene (r=-0.75, P=0.03, n=8; see Figure 24), the FCGR1B (Fc fragment of IgG, high affinity Ib, receptor (CD64)) gene (r=-0.80, P=0.017, n=8), the DRD2 (D(2) dopamine receptor) gene (i=0.91, P=0.002, n=8) and the SOD1 (superoxide dismutase) gene (r=-0.74, P=0.038).

**Table 10**

| | |
|---|---|
| ACTC1 | Cardiac muscle alpha actin 1 |
| AK090950 | KIAA1345 protein |
| CIB4 | Calcium and integrin binding family member 4 |
| FCGR1 B | Fc fragment of IgG, high affinity Ib, receptor (CD64) |
| HSP90AB3P | Heat shock protein 90kDa alpha (cytosolic), class B member 3 (pseudogene) |
| IGF1 R | Insulin-like growth factor 1 receptor |
| LOC401027 | Hypothetical protein LOC401027 |
| MGC48628 | Similar to KIAA1680 protein (=hypothetical protein LOC401145) |
| PPARA | Peroxisome proliferator-activated receptor alpha |
| RAI16 | Retinoic acid induced 16 |
| SOD1 | Superoxide dismutase 1, soluble (amyotrophic lateral sclerosis 1 (adult)) |
| TRPC1 | Transient receptor potential cation channel, subfamily C, member 1 |
| VCAM1 | Vascular cell adhesion molecule 1 |
| CDK10 | cyclin-dependent kinase (CDC2-like) 10 |
| COMT | catechol-O-methyltransferase |
| CREB1 | cAMP responsive element binding protein 1 |
| DRD2 | D(2) dopamine receptor |
| FADS2 | fatty acid desaturase 2 (delta-6-desaturase) |
| FOS | v-fos FBJ murine osteosarcoma viral oncogene Homolog |
| HSFY2 | HSFY2 heat shock transcription factor, Y linked 2 (more telomeric copy) |
| IGFBP1 | insulin-like growth factor binding protein 1 |
| OTX1 | orthodenticle homeobox 1 |
| PGF | placental growth factor, vascular endothelial growth factor-related protein |
| RXRB | retinoid X receptor, beta |
| S100B | S100 calcium binding protein B |
| SLC6A4 | solute carrier family 6 (neurotransmitter transporter, serotonin), member 4 (5-HTT) |
| SYN1 | synapsin I |

**Appendix 1 Cardiovascular/bone candidate genes examined in preliminary analyses**

| | | |
|---|---|---|
| Official symbol | Gene name | MIM, GeneID |
| ACTC1 | Cardiac muscle alpha actin 1 | MIM: 102540, GeneID: 70 |
| AGTR1 | Angiotensin II receptor, type 1 | MIM: 106165, GeneID: 185 |
| ATP2A3 | ATPase, Ca++ transporting, ubiquitous (=PMCA3) | MIM: 601929, GeneID: 489 |
| ATP2B1 | ATPase, Ca++ transporting, plasma membrane 1 (=PMCA1) | MIM: 108731, GeneID: 490 |
| EDN1 | Endothelin 1 | MIM: 131240, GeneID: 1906 |
| ESR1 | Estrogen receptor 1 (alpha) | MIM: 133430, GeneID: 2099 |
| HSD11 B2 | Hydroxysteroid (11-beta) dehydrogenase 2 | MIM: 218030, GeneID: 3291 |
| IGF1 R | Insulin-like growth factor 1 receptor | MIM: 147370, GeneID: 3480 |
| IL1A | Interleukin 1, alpha | MIM: 147760, GeneID: 3552 |
| IL8 | Interleukin 8 | MIM: 146930, GeneID: 3576 |
| | Matrix metallopeptidase 2 (gelatinase A, 72kDa gelatinase, 72kDa | |
| MMP2 | type IV collagenase) | MIM: 120360, GeneID: 4313 |
| | Matrix metallopeptidase 9 (gelatinase B, 92kDa gelatinase, 92kDa | MIM: 120361, GeneID: 4318 |
| MMP9 | type IV collagenase) | |
| MYH11 | Smooth muscle myosin heavy chain 11 | MIM: 160745, GeneID: 4629 |
| MYOCD | Myocardin | MIM: 606127, GeneID: 93649 |
| NOS3 | Nitric oxide synthase 3 (endothelial cell) (eNOS) | MIM: 163729, GeneID: 4846 |
| NOX5 | NADPH oxidase, EF-hand calcium binding domain 5 | MIM: 606572, GeneID: 79400 |
| | Glucocorticoid receptor; nuclear receptor subfamily 3, group C, | |
| NR3C1 | member 1 | MIM: 138040, GeneID: 2908 |
| PPARA | Peroxisome proliferator-activated receptor alpha | MIM: 170998, GeneID: 5465 |
| PPARG | Peroxisome proliferator-activated receptor gamma | MIM: 601487, GeneID: 5468 |
| | Superoxide dismutase 1, soluble (amyotrophic lateral sclerosis 1 | |
| SOD1 | (adult)) | MIM: 147450, GeneID: 6647 |
| TAGLN | Transgelin (=Smooth muscle protein 22-alpha) | MIM: 600818, GeneID: 6876 |
| TNF | Tumor necrosis factor (TNF superfamily, member 2)(TNFa) | MIM: 191160, GeneID: 7124 |
| VCAM1 | Vascular cell adhesion molecule 1 | MIM: 192225, GeneID: 7412 |
| VEGFA | Vascular endothelial growth factor A | MIM: 192240, GeneID: 7422 |

**Appendix 2 Gene promoters with greatest inter-subject variation in methylation**

| **Official symbol** | **Gene name** | **MIM**, **GeneID** / **Reporter** |
|---|---|---|
| (/description) | | |
| ADAR | Adenosine deaminase, RNA-specific | MIM: 601059, GeneID: 103 |
| AK090950 | KIAA1345 protein | AK090950 |
| AK128539 | Homo sapiens cDNA FLJ46698 fis, clone TRACH3013684 | AK128539 |
| ANXA4 | Annexin A4 | MIM: 106491, GeneID:307 |
| BDP1 | B double prime 1, subunit of RNA polymerase III transcription initiation factor IIIB | MIM: 607012, GeneID: 55814 |
| C1orf185 | Chromosome 1 open reading frame 185 | GeneID:284546 |
| CIB4 | Calcium and integrin binding family member 4 | MIM: 610646, GeneID: 130106 |
| COX6C | Cytochrome c oxidase subunit Vic | AK128382 |
| DEFB107A | Defensin, beta 107A | GeneID:245910 |
| ECHDC2 | Enoyl Coenzyme A hydratase domain containing 2 | BX647186 |
| FCGR1 B | Fc fragment of IgG, high affinity Ib, receptor (CD64) | MIM: 601502, GeneID: 2210 |
| FLAD1 | FAD1 flavin adenine dinucleotide synthetase homolog | MIM: 610595, GeneID: 80308 |
| FLJ13231 | Hypothetical protein FLJ13231 | GeneID:65250 |
| FLJ23577 | KPL2 protein | MIM: 610172, GeneID: 79925 |
| FLJ41821 | FLJ41821 protein | GeneID:401011 |
| GLO1 | Glyoxalase I | MIM: 138750, GeneID: 2739 |
| HEMGN | Hemogen | MIM: 610715, GeneID: 55363 |
| HGSNAT | Heparan-alpha-glucosaminide N-acetyltransferase | MIM: 610453, GeneID: 138050 |
| HIVEP2 | HIV type I enhancer binding protein 2 | MIM: 143054, GeneID: 3097 |
| HSP90AB3P | Heat shock protein 90kDa alpha (cytosolic), class B member 3 (pseudogene) | GeneID:3327 |
| hTAK1 | Human nuclear receptor hTAK1 | U10990 |
| IL6ST | Interleukin 6 signal transducer (gp130, oncostatin M receptor) | AB102799 |
| ISG15 | ISG15 ubiquitin-like modifier | MIM: 147571, GeneID: 9636 |
| KLHL5 | Kelch-like 5 (Drosophila) | MIM: 608064,GeneID: 51088 |
| LOC401027 | Hypothetical protein LOC401027 | GeneID: 401027 |
| LOC644936 | Similar to cytoplasmic beta-actin | GeneID: 644936 |
| LOC645156 | Hypothetical protein LOC645156 | GeneID: 645156 |
| LOC646870 | Hypothetical protein LOC646870 | GeneID: 646870 |
| MCL1 | Myeloid cell leukemia sequence 1 (BCL2-related) | MIM: 159552, GeneID: 4170 |
| MGC12538 | Hypothetical protein MGC12538 | GeneID: 84832 |
| MGC48628 | Similar to KIAA1680 protein (=hypothetical protein LOC401145) NADH dehydrogenase (ubiquinone) 1 alpha subcomplex, 5, | GeneID: 401145 |
| NDUFA5 | 13kDa | MIM: 601677, GeneID: 4698 |
| OGDH | Oxoglutarate (alpha-ketoglutarate) dehydrogenase (lipoamide) | MIM: 203740, GeneID: 4967 |
| OR2G2 | Olfactory receptor, family 2, subfamily G, member 2 | GeneID: 81470 |
| OR5C1 | Olfactory receptor, family 5, subfamily C, member 1 | GeneID: 392391 |
| PCDH1 | Protocadherin 1 (cadherin-like 1) | MIM: 603626, GeneID: 5097 |
| PIGK | Phosphatidylinositol glycan anchor biosynthesis, class K | MIM: 605087, GeneID: 10026 |
| PIK3CD | Phosphoinositide-3-kinase, catalytic, delta polypeptide | MIM: 602839, GeneID: 5293 |
| POLQ | Polymerase (DNA directed), theta | MIM: 604419, GeneID: 10721 |
| PREB | Prolactin regulatory element binding | MIM: 606395, GeneID: 10113 |
| PSME4 | Proteasome (prosome, macropain) activator subunit 4 | MIM: 607705, GeneID: 23198 |
| QSCN6 | Quiescin Q6 | MIM: 603120, GeneID: 5768 |
| RAI16 | Retinoic acid induced 16 | GeneID: 64760 |
| RALGPS1 | Ral GEF with PH domain and SH3 binding motif 1 | GeneID: 9649 |
| RC3H2 | Ring finger and CCCH-type zinc finger domains 2 | GeneID: 54542 |
| RTN4 | Reticulon 4 | MIM: 604475, GeneID: 57142 |
| SDHALP2 | Succinate dehydrogenase complex, subunit A, flavoprotein pseudogene 2 | GeneID: 727956 |
| TPM3 | Tropomyosin 3 | MIM: 191030, GeneID: 7170 |
| TRPC1 | Transient receptor potential cation channel, subfamily C, member 1 | MIM: 602343, GeneID: 7220 |
| TSN | Translin | MIM: 600575,GeneID: 7247 |

**Appendix 3 Additional candidate genes examined**

| | | |
|---|---|---|
| Human gene | Human gene name | MIM, GeneID/Reporter |
| symbol | | |
| CDK10 | cyclin-dependent kinase (CDC2-like) 10 | MIM: 603464, GeneID: 8558 |
| CDK4 | cyclin-dependent kinase 4 | MIM: 123829, GeneID: 1019 |
| CDK9 | cyclin-dependent kinase 9 (CDC2-related kinase) | MIM: 603251, GeneID: 1025 |
| CDKN1A | cyclin-dependent kinase inhibitor 1A (p21, Cip1) | MIM: 116899, GeneID: 1026 |
| CDKN2A | cyclin-dependent kinase inhibitor 2A (melanoma, p16, inhibits CDK4) | MIM: 600160, GeneID: 1029 |
| CDKN2B | cyclin-dependent kinase inhibitor 2B (p15, inhibits CDK4) | MIM: 600431, GeneID: 1030 |
| CDKN2C | cyclin-dependent kinase inhibitor 2C (p18, inhibits CDK4) | MIM: 603369, GeneID: 1031 |
| CHRM1 | cholinergic receptor, muscarinic 1 | MIM: 118510, GeneID: 1128 |
| COMT | catechol-O-methyltransferase | MIM: 116790, GeneID: 1312 |
| CREB1 | cAMP responsive element binding protein 1 | MIM: 123810, GeneID: 1385 |
| DRD1 IP | dopamine receptor D1 interacting protein | MIM: 604647, GeneID: 50632 |
| DRD2 | D(2) dopamine receptor | MIM: 126450, GeneID: 1813 |
| DRD3 | dopamine receptor D3 | MIM: 126451, GeneID: 1814 |
| DRD4 | Dopamine receptor D4 | MIM: 126452, GeneID: 1815 |
| EGR1 | early growth response 1 | MIM: 128990, GeneID: 1958 |
| FADS1 | fatty acid desaturase 1 (delta-5 desaturase) | MIM: 606148, GeneID: 3992 |
| FADS2 | fatty acid desaturase 2 (delta-6-desaturase) | MIM: 606149, GeneID: 9415 |
| FADS3 | fatty acid desaturase 3 (delta-9-desaturase) | MIM: 606150, GeneID: 3995 |
| FAF1 | Fas (TNFRSF6) associated factor 1 | MIM: 604460, GeneID: 11124 |
| FLT1 | fms-related tyrosine kinase 1 (VEGF/vascular permeability factor receptor) | MIM: 165070, GeneID: 2321 |
| FOS | v-fos FBJ murine osteosarcoma viral oncogene homolog | MIM: 164810, GeneID: 2353 |
| FTO | fat mass and obesity associated | GeneID: 79068 |
| GABRB1 | gamma-aminobutyric acid (GABA) A receptor, beta 1 | MIM: 137190, GeneID: 2560 |
| GABRB3 | gamma-aminobutyric acid (GABA) A receptor, beta 3 | MIM: 137192, GeneID: 2562 |
| GAD1 | glutamate decarboxylase 1 (brain, 67kDa) | MIM: 605363, GeneID: 2571 |
| GRIK2 | glutamate receptor, ionotropic, kainate 2 | MIM: 138244, GeneID: 2898 |
| GRIN3B | glutamate receptor, N-methyl-D-aspartate 3B (NMDA receptor subunit 3B) | MIM: 606651, GeneID: 116444 |
| HSFY1 | heat shock transcription factor, Y-linked 1 (more centromeric copy) | MIM: 400029, GeneID: 86614 |
| HSFY2 | HSFY2 heat shock transcription factor, Y linked 2 (more telomeric copy) | GeneID: 159119 |
| HTR1A | 5-hydroxytryptamine (serotonin) receptor 1A | MIM: 109760, GeneID: 3350 |
| HTR2A | 5-hydroxytryptamine (serotonin) receptor 2A | MIM: 182135, GeneID: 3356 |
| HTR4 | 5-hydroxytryptamine (serotonin) receptor 4 | MIM: 602164, GeneID: 3360 |
| IGFBP1 | insulin-like growth factor binding protein 1 | MIM: 146730, GeneID: 3484 |
| MAOA | monoamine oxidase A | MIM: 309850, GeneID: 4128 |
| MAPK1 | mitogen-activated protein kinase 1 (protein tyrosine kinase ERK2) | MIM: 176948, GeneID: 5594 |
| MYC | v-myc myelocytomatosis viral oncogene homolog (avian) | MIM: 190080, GeneID: 4609 |
| NCAM1 | neural cell adhesion molecule 1 | MIM: 116930, GeneID: 4684 |
| NTRK2 | neurotrophic tyrosine kinase, receptor, type 2 | MIM: 600456, GeneID: 4915 |
| OTX1 | orthodenticle homeobox 1 | MIM: 600036, GeneID: 5013 |
| PGF | placental growth factor, vascular endothelial growth factor-related protein | MIM: 601121, GeneID: 5228 |
| PPARD | peroxisome proliferator-activated receptor delta | MIM: 600409, GeneID: 5467 |
| PRKCA | protein kinase C, alpha | MIM: 176960, GeneID: 5578 |
| RELN | reelin | MIM: 600514, GeneID: 5649 |
| RXRA | retinoid X receptor, alpha | MIM: 180245, GeneID: 6256 |
| RXRB | retinoid X receptor, beta | MIM: 180246, GeneID: 6257 |
| RXRG | retinoid X receptor, gamma | MIM: 180247, GeneID: 6258 |
| S100B | S100 calcium binding protein B | MIM: 176990, GeneID: 6285 |
| SCD | stearoyl-CoA desaturase (delta-9-desaturase) | MIM: 604031, GeneID: 6319 |
| SEMA4F | sema domain, Ig domain, TM domain & short cytoplasmic domain, (semaphorin) 4F | MIM: 603706, GeneID: 10505 |
| SLC6A3 | Sodium-dependent dopamine transporter (DA transporter) (DAT) | MIM: 126455, GeneID: 6531 |
| SLC6A4 | solute carrier family 6 (neurotransmitter transporter, serotonin), member 4 (5-HTT) | MIM: 182138, GeneID: 6532 |
| SYN1 | synapsin I | MIM: 313440, GeneID: 6853 |
| SYN2 | synapsin II | MIM: 600755, GeneID: 6854 |
| SYN3 | synapsin III | MIM: 602705, GeneID: 8224 |
| TNFRSF1A | tumor necrosis factor receptor superfamily, member 1A | MIM: 191190, GeneID: 7132 |

## Claims

1. A method of predicting a phenotypic characteristic of a human or non-human animal, said phenotypic characteristic being selected from:
(i) total body lean mass and/or percentage lean mass, or susceptibility for sarcopenia or impaired muscle function;
(ii) impaired skeletal development associated with reduced bone mineral content or a disease condition associated with low bone mineral content such as osteoporosis;
and said method comprising determining the degree of methylation of the promoter of the Retinoid X receptor, alpha (RXRA) gene or a combination of the promoter of the RXRA gene and at least one other gene in a perinatal tissue sample, wherein the degree of said methylation of the gene or genes of interest correlates with propensity for said phenotypic characteristic.

2. A method as claimed in claim 1 wherein said tissue sample is umbilical cord.

3. A method as claimed in claim 1 wherein said sample is adipose tissue.

4. A method as claimed in any one of claims 2 or 3 for predicting total body lean mass and /or percentage lean mass and susceptibility for sarcopenia or impaired muscle function.

5. A method as claimed in claim 4 wherein methylation of the promoter of the RXRA gene in combination with the methylation of the promoter of at least one other gene is determined, wherein the other gene is selected from the genes listed in the following Table 2:
**Table 2**
| | |
|---|---|
| AK090950 | KIAA1345 protein |
| ATP2A3 | ATPase, Ca++ transporting, ubiquitous (=PMCA3) |
| BDP1 | B double prime 1, subunit of RNA polymerase III transcription initiation factor IIIB |
| C1orf185 | Chromosome 1 open reading frame 185 |
| CIB4 | Calcium and integrin binding family member 4 |
| COX6C | Cytochrome c oxidase subunit Vic |
| DEFB107A | Defensin, beta 107A |
| ECHDC2 | Enoyl Coenzyme A hydratase domain containing 2 |
| FCGR1B | Fc fragment of IgG, high affinity Ib, receptor (CD64) |
| FLAD1 | FAD1 flavin adenine dinucleotide synthetase homolog |
| FLJ41821 | FLJ41821 protein |
| HGSNAT | Heparan-α-glucosaminide N-acetyltransferase |
| HIVEP2 | HIV type I enhancer binding protein 2 |
| HSD11B2 | Hydroxysteroid (11-beta) dehydrogenase 2 |
| IL8 | Interleukin-8 |
| ISG15 | ISG15 ubiquitin-like modifier |
| KLHL5 | Kelch-like 5 (Drosophila) |
| LOC401027 | Hypothetical protein LOC401027 |
| MGC48628 | Similar to KIAA1680 protein (=hypothetical protein LOC401145) |
| MMP2 | Matrix metalloproteinase-2 |
| MYH11 | Smooth muscle myosin heavy chain 11 |
| NOS3 | Nitric oxide synthase 3 (endothelial cell) (eNOS) |
| NR3C1 | Glucocorticoid receptor; nuclear receptor subfamily 3, group C, member 1 |
| OGDH | Oxoglutarate (alpha-ketoglutarate) dehydrogenase (lipoamide) |
| OR5C1 | Olfactory receptor, family 5, subfamily C, member 1 |
| PIGK | Phosphatidylinositol glycan anchor biosynthesis, class K |
| PIK3CD | Phosphoinositide-3-kinase, catalytic, delta polypeptide |
| PREB | Prolactin regulatory element binding |
| RAI16 | Retinoic acid induced 16 |
| RALGPS1 | Ral GEF with PH domain and SH3 binding motif 1 |
| RC3H2 | Ring finger and CCCH-type zinc finger domains 2 |
| CDK9 | cyclin-dependent kinase 9 (CDC2-related kinase) |
| CDKN2A | cyclin-dependent kinase inhibitor 2A (melanoma, p16, inhibits CDK4) |
| CHRM1 | cholinergic receptor, muscarinic 1 |
| COMT | catechol-O-methyltransferase |
| DRD3 | dopamine receptor D3 |
| EGR1 | early growth response 1 |
| FADS1 | fatty acid desaturase 1 (delta-5 desaturase) |
| FADS2 | fatty acid desaturase 2 (delta-6-desaturase) |
| FADS3 | fatty acid desaturase 3 (delta-9-desaturase) |
| FAF1 | Fas (TNFRSF6) associated factor 1 |
| FLT1 | fms-related tyrosine kinase 1 (VEGF/vascular permeability factor receptor) |
| FOS | v-fos FBJ murine osteosarcoma viral oncogene homolog |
| FTO | fat mass and obesity associated |
| GABRB1 | gamma-aminobutyric acid (GABA) A receptor, beta 1 |
| GRIK2 | glutamate receptor, ionotropic, kainate 2 |
| GRIN3B | glutamate receptor, N-methyl-D-aspartate 3B (NMDA receptor subunit 3B) |
| HTR2A | 5-hydroxytryptamine (serotonin) receptor 2A |
| IGFBP1 | insulin-like growth factor binding protein 1 |
| MAOA | monoamine oxidase A |
| MAPK1 | mitogen-activated protein kinase 1 (protein tyrosine kinase ERK2) |
| MYC | v-myc myelocytomatosis viral oncogene homolog (avian) |
| OTX1 | orthodenticle homeobox 1 |
| RXRB | retinoid X receptor, beta |
| S100B | S100 calcium binding protein B |
| SCD | stearoyl-CoA desaturase (delta-9-desaturase) |
| SEMA4F | sema domain, Ig domain, TM domain & short cytoplasmic domain, (semaphorin) 4F |
| SLC6A3 | Sodium-dependent dopamine transporter (DA transporter) (DAT) |
| SLC6A4 | solute carrier family 6 (neurotransmitter transporter, serotonin), member 4 (5-HTT) |

6. A method as claimed in any one of claims 2 or 3 for predicting impaired skeletal development associated with reduced bone mineral content and propensity for disease conditions associated with low bone mineral content such as osteoporosis.

7. A method as claimed in claim 6 wherein methylation of the promoter of the RXRA gene in combination with the methylation of the promoter of at least one other gene is determined, wherein the other gene is selected from the genes listed in the following Table 3:
**Table 3**
| | |
|---|---|
| DEFB107A | Defensin, beta 107A |
| ECHDC2 | Enoyl Coenzyme A hydratase domain containing 2 |
| FLAD1 | FAD1 flavin adenine dinucleotide synthetase homolog |
| FLJ23577 | KPL2 protein |
| HGSNAT | Heparan-alpha-glucosaminide N-acetyltransferase |
| LOC646870 | Hypothetical protein LOC646870 |
| MCL1 | Myeloid cell leukemia sequence 1 (BCL2-related) |
| MMP9 | Matrix metallopeptidase 9 (gelatinase B, 92kDa gelatinase, 92kDa type IV collaqenase) |
| NOS3 | Nitric oxide synthase 3 (endothelial cell) (eNOS) |
| PI3KCD | Phosphoinositide-3-kinase, catalytic, delta polypeptide |
| PPARG | Peroxisome proliferator-activated receptor gamma |
| PREB | Prolactin regulatory element binding |
| RALGPS1 | Ral GEF with PH domain and SH3 binding motif 1 |
| TNF | Tumor necrosis factor (TNF superfamily, member 2)(TNFa) |
| TSN | Translin |
| VEGFA | Vascular endothelial growth factor A |
| CDK9 | cyclin-dependent kinase 9 (CDC2-related kinase) |
| CDKN1A | cyclin-dependent kinase inhibitor 1A (p21, Cip1) |
| CDKN2A | cyclin-dependent kinase inhibitor 2A (melanoma, p16, inhibits CDK4) |
| CDKN2B | cyclin-dependent kinase inhibitor 2B (p15, inhibits CDK4) |
| CDKN2C | cyclin-dependent kinase inhibitor 2C (p18, inhibits CDK4) |
| EGR1 | early growth response 1 |
| FADS3 | fatty acid desaturase 3 (delta-9-desaturase) |
| FAF1 | Fas (TNFRSF6) associated factor 1 |
| FLT1 | fms-related tyrosine kinase 1 (VEGF/vascular permeability factor receptor) |
| GAD1 | glutamate decarboxylase 1 (brain, 67kDa) |
| GRIK2 | glutamate receptor, ionotropic, kainate 2 |
| IGFBP1 | insulin-like growth factor binding protein 1 |
| MAOA | monoamine oxidase A |
| MYC | v-myc myelocytomatosis viral oncogene homolog (avian) |
| RXRB | retinoid X receptor, beta |
| SCD | stearoyl-CoA desaturase (delta-9-desaturase) |
| SEMA4F | sema domain, Ig domain, TM domain & short cytoplasmic domain, (semaphorin) 4F |
| SLC6A4 | solute carrier family 6 (neurotransmitter transporter, serotonin), member 4 (5-HTT) |

8. A kit comprising primers and methylation-sensitive restriction enzymes for use in carrying out methylation-sensitive amplification of a combination of the promoter of the RXRA gene and the promoter of at least one other gene for predicting one or more phenotypic characteristics in accordance with claim 1, wherein the other gene is selected from the genes listed in Tables 2 and 3 of claims 5, and 7, respectively.

## Patentansprüche

1. Verfahren zur Vorhersage eines phänotypischen Merkmals eines menschlichen oder nichtmenschlichen Lebewesens, wobei das phänotypische Merkmal ausgewählt wird aus:
(i) Gesamtkörpermagermasse und/oder prozentualer Magermasse, oder Anfälligkeit für Sarkopenie oder beeinträchtige Muskelfunktion;
(ii) beeinträchtigter Skelettentwicklung, assoziiert mit reduziertem Knochenmineralgehalt, oder einem Erkrankungszustand, assoziiert mit geringem Knochenmineralgehalt, wie Osteoporose;
und wobei das Verfahren Bestimmen des Grads an Methylierung des Promoters des Retinoid-X-Rezeptor-alpha- (RXRA-) Gens oder einer Kombination von dem Promoter des RXRA-Gens und zumindest einem anderen Gen in einer perinatalen Gewebeprobe umfasst, wobei der Grad der Methylierung des Gens oder der Gene von Interesse mit der Neigung zum phänotypischen Merkmal korreliert.

2. Verfahren nach Anspruch 1, wobei die Gewebeprobe Nabelschnur ist.

3. Verfahren nach Anspruch 1, wobei die Probe Fettgewebe ist.

4. Verfahren nach einem der Ansprüche 2 oder 3 zur Vorhersage der Gesamtkörpermagermasse und/oder der prozentualen Magermasse und der Anfälligkeit für Sarkopenie oder beeinträchtigte Muskelfunktion.

5. Verfahren nach Anspruch 4, wobei die Methylierung des Promoters des RXRA-Gens in Kombination mit der Methylierung des Promoters zumindest eines anderen Gens bestimmt wird, wobei das andere Gen aus den Genen ausgewählt wird, die in der folgenden Tabelle 2 aufgelistet sind:
**Tabelle 2**
| | |
|---|---|
| AK090950 | KIAA1345-Protein |
| ATP2A3 | ATPase, Ca⁺⁺-transportierend, ubiquitär (=PMCA3) |
| BDP1 | B double prime 1, Untereinheit von RNA-Polymerase-III-Transkriptionsinitiationsfaktor IIIB |
| C1orf185 | Chromosom 1 open reading frame 185 |
| CIB4 | Mitglied 4 der Calcium und Integrin bindenden Familie |
| OX6C | Cytochrom-c-Oxidase-Untereinheit VIc |
| DEFB107A | Defensin, beta 107A |
| ECHDC2 | Enoyl-Coenzym-A-Hydratase-Domäne enthaltendes 2 |
| FCGR1B | Fc-Fragment von IgG, hohe Affinität Ib, Rezeptor (CD64) |
| FLAD1 | FAD1, Flavin-Adenin-Dinukleotid-Synthetase, Homolog |
| FLJ41821 | FLJ41821-Protein |
| HGSNAT | Heparan-α-Glucosaminid-N-Acetyltransferase |
| HIVEP2 | HIV-Typ-I-Enhancer-bindendes Protein 2 |
| HSD11B2 | Hydroxysteroid- (11-beta) Dehydrogenase 2 |
| IL8 | Interleukin-8 |
| ISG15 | Ubiquitin-ähnlicher Modifizierer ISG15 |
| KLHL5 | Kelch-like 5 (Drosophila) |
| LOC401027 | hypothetisches Protein LOC401027 |
| MGC48628 | ähnlich dem KIAA1680-Protein (=hypothetisches Protein LOC401145) |
| MMP2 | Matrix-Metalloproteinase-2 |
| MYH11 | Glattmuskel-Myosin-Schwerkette 11 |
| NOS3 | Stickoxid-Synthase 3 (Endothelzelle) (eNOS) |
| NR3C1 | Glukokortikoid-Rezeptor; Kernrezeptor-Unterfamilie 3, Gruppe C, Mitglied 1 |
| OGDH | Oxoglutarat- (alpha-Ketoglutarat-) Dehydrogenase (Lipoamid) |
| OR5C1 | tolfaktorischer Rezeptor, Familie 5, Unterfamilie C, Mitglied 1 |
| PIGK | Phosphatidylinositol-Glycan-Anker-Biosynthese, Klasse K |
| PIK3CD | Phosphoinositid-3-Kinase, katalytisch, delta-Polypeptid |
| PREB | Prolactin-Regulatorelement bindend |
| RAI16 | Retinsäure-induziertes 16 |
| RALGPS1 | Ral GEF mit PH-Domäne und SH3-Bindungsmotiv 1 |
| RC3H2 | Ringfinger- und CCCH-Typ-Zinkfinger-Domänen 2 |
| DK9 | Cyclin-abhängige Kinase 9 (CDC2-verwandte Kinase) |
| DKN2A | Cyclin-abhängiger Kinase-Hemmer 2A (Melanom, p16, hemmt CDK4) |
| CHRM1 | cholinerger Rezeptor, muscarinisch 1 |
| COMT | Catechol-O-Methyltransferase |
| DRD3 | Dopamin-Rezeptor D3 |
| EGR1 | Early Growth Response 1 |
| FADS1 | Fettsäure-Desaturase 1 (delta-5-Desaturase) |
| FADS2 | Fettsäure-Desaturase 2 (delta-6-Desaturase) |
| FADS3 | Fettsäure-Desaturase 3 (delta-9-Desaturase) |
| FAF1 | Fas- (TNFRSF6-) assoziierter Faktor 1 |
| FLT1 | fms-verwandte Tyrosinkinase 1 (VEGF/Gefäßpermeabilitätsfaktor-Rezeptor) |
| FOS | v-fos FBJ-Maus-Osteosarkom-Virusonkogen-Homolog |
| FrO | Fettmasse- und Obesitas-assoziiert |
| GABRB1 | gamma-Aminobuttersäure- (GABA-) A-Rezeptor, beta 1 |
| GRIK2 | Glutamat-Rezeptor, ionotrop, Kainat 2 |
| GRIN3B | Glutamat-Rezeptor, N-Methyl-D-Aspartat 3B (NMDA-Rezeptor-Untereinheit 3B) |
| HTR2A | 5-Hydroxytryptamin- (Serotonin-) Rezeptor 2A |
| IGFBP1 | insulinähnlichen Wachstumsfaktor bindendes Protein 1 |
| MAOA | Monoamin-Oxidase A |
| MAPK1 | Mitogen-aktivierte Proteinkinase 1 (Proteintyrosinkinase ERK2) |
| MYC | v-myc, Myelocytomatose-Virusonkogen, Homolog (Vogel) |
| OTX1 | Orthodenticle-Homöobox 1 |
| RXRB | Retinoid-X-Rezeptor, beta |
| S100B | calciumbindendes S100-Protein B |
| SCD | Stearoyl-CoA-Desaturase (delta-9-Desaturase) |
| SEMA4F | Sema-Domäne, Ig-Domäne, TM-Domäne & kurze cytoplasmatische Domäne, (Semaphorin) 4F |
| SLC6A3 | Natrium-abhängiger Dopamin-Transporter (DA-Transporter) (DAT) |
| SLC6A4 | Solute-Carrier-Familie 6 (Neurotransmitter-Transporter, Serotonin), Mitglied 4 (5-HTT) |

6. Verfahren nach einem der Ansprüche 2 oder 3 zur Vorhersage von beeinträchtigter Skelettentwicklung, assoziiert mit reduziertem Knochenmineralgehalt, und Neigung zu Erkrankungszuständen, assoziiert mit geringem Knochenmineralgehalt, wie Osteoporose.

7. Verfahren nach Anspruch 6, wobei die Methylierung des Promoters des RXRA-Gens in Kombination mit der Methylierung des Promoters zumindest eines anderen Gens bestimmt wird, wobei das andere Gen aus den Genen ausgewählt wird, die in der folgenden Tabelle 3 aufgelistet sind:
**Tabelle 3**
| | |
|---|---|
| DEFB107A | Defensin, beta 107A |
| ECHDC2 | Enoyl-Coenzym-A-Hydratase-Domäne enthaltendes 2 |
| FLAD1 | FAD1, Flavin-Adenin-Dinukleotid-Synthetase, Homolog |
| FLJ23577 | KPL2-Protein |
| HGSNAT | Heparan-alpha-Glucosaminid-N-Acetyltransferase |
| LOC646870 | hypothetisches Protein LOC646870 |
| MCL1 | Myeloische-Zellen-Leukämie-Sequenz 1 (BCL2-verwandt) |
| MMP9 | Matrix-Metallopeptidase 9 (Gelatinase B, 92 kDa Gelatinase, 92 kDa Typ-IV-Collagenase) |
| NOS3 | Stickoxid-Synthase 3 (Endothelzelle) (eNOS) |
| PI3KCD | Phosphoinositid-3-Kinase, katalytisch, delta-Polypeptid |
| PPARG | Peroxisomen-Proliferator-aktivierter Rezeptor gamma |
| PREB | Prolactin-Regulatorelement bindend |
| RALGPS1 | Ral GEF mit PH-Domäne und SH3-Bindungsmotiv 1 |
| TNF | Tumornekrosefaktor (TNF-Superfamilie, Mitglied 2) (TNFa) |
| TSN | Translin |
| VEGFA | vaskulärer Endothelwachstumsfaktor A |
| DK9 | Cyclin-abhängige Kinase 9 (CDC2-verwandte Kinase) |
| DKN1A | Cyclin-abhängiger Kinase-Hemmer 1A (p21, Cip1) |
| DKN2A | Cyclin-abhängiger Kinase-Hemmer 2A (Melanom, p16, hemmt CDK4) |
| DKN2B | Cyclin-abhängiger Kinase-Hemmer 2B (p15, hemmt CDK4) |
| CDKN2C | Cyclin-abhängiger Kinase-Hemmer 2C (p18, hemmt CDK4) |
| EGR1 | Early Growth Response 1 |
| FADS3 | Fettsäure-Desaturase 3 (delta-9-Desaturase) |
| FAF1 | Fas- (TNFRSF6-) assoziierter Faktor 1 |
| FLT1 | fms-verwandte Tyrosinkinase 1 (VEGF/Gefäßpermeabilitätsfaktor-Rezeptor) |
| GAD1 | Glutamat-Decarboxylase 1 (Gehirn, 67 kDa) |
| GRIK2 | Glutamat-Rezeptor, ionotrop, Kainat 2 |
| IGFBP1 | insulinähnlichen Wachstumsfaktor bindendes Protein 1 |
| MAOA | Monoamin-Oxidase A |
| MYC | v-myc, Myelocytomatose-Virusonkogen, Homolog (Vogel) |
| RXRB | Retinoid-X-Rezeptor, beta |
| SCD | Stearoyl-CoA-Desaturase (delta-9-Desaturase) |
| SEMA4F | Sema-Domäne, Ig-Domäne, TM-Domäne & kurze cytoplasmatische Domäne, (Semaphorin) 4F |
| SLC6A4 | Solute-Carrier-Familie 6 (Neurotransmitter-Transporter, Serotonin), Mitglied 4 (5-HTT) |

8. Kit, umfassend Primer und methylierungssensitive Restriktionsenzyme zur Verwendung bei Ausführung methylierungssensitiver Amplifikation einer Kombination von dem Promoter des RXRA-Gens und dem Promoter zumindest eines anderen Gens zwecks Vorhersage eines oder mehrerer phänotypischer Merkmale gemäß Anspruch 1, wobei das andere Gen aus den Genen ausgewählt wird, die in Tabelle 2 und 3 von Anspruch 5 bzw. 7 aufgelistet sind.

## Revendications

1. Méthode pour la prédiction d'une caractéristique phénotypique d'un humain ou d'un animal non humain, ladite caractéristique phénotypique étant choisie parmi:
(i) la masse maigre corporelle totale et/ou le pourcentage de masse maigre ou la susceptibilité pour une sarcopénie ou une fonction musculaire détériorée;
(ii) un développement squelettique détérioré associé à un contenu minéral osseux réduit ou un état pathologique associé à un contenu minéral osseux bas tel qu'une ostéoporose;
et ladite méthode comprenant la détermination du degré de méthylation du promoteur du gène récepteur X des rétinoïdes, alpha (RXRA) ou d'une combinaison du promoteur du gène RXRA et d'au moins un autre gène dans un échantillon de tissu périnatal, où le degré de ladite méthylation du gène ou des gènes intéressants est en corrélation avec la propension pour ladite caractéristique phénotypique.

2. Méthode selon la revendication 1, dans laquelle ledit échantillon de tissu est un cordon ombilical.

3. Méthode selon la revendication 1, dans laquelle ledit échantillon est un tissu d' adipose.

4. Méthode selon l'une quelconque des revendications 2 ou 3 pour la prédiction de la masse maigre corporelle totale et/ou du pourcentage de masse maigre ou d'une susceptibilité pour une sarcopénie ou une fonction musculaire détériorée.

5. Méthode selon la revendication 4, dans laquelle la méthylation du promoteur du gène RXRA en combinaison avec la méthylation du promoteur d'au moins un autre gène est déterminée, dans laquelle l'autre gène est choisi parmi les gènes énumérés dans le Tableau 2 suivant:
**Tableau 2**
| | |
|---|---|
| AK090950 | protéine KIAA1345 |
| ATP2A3 | ATPase, transportant Ca++, omniprésente (=PMCA3) |
| BDP1 | B double prime 1, sous-unité d'ARN polymérase III facteur d'initiation de transcription IIIB |
| C1orf185 | cadre de lecture ouvert 185 du chromosome 1 |
| CIB4 | membre 4 de la famille de liaison de calcium et d'intégrine |
| COX6C | sous-unité Vic de cytochrome c oxydase |
| DEFB107A | défensine, bêta 107A |
| ECHDC2 | domaine d'énoyl-coenzyme A hydratase contenant 2 |
| FCGR1B | fragment Fc de IgG, haute affinité Ib, récepteur (CD64) |
| FLAD1 | homologue de FAD1 flavine adénine dinucléotide synthétase |
| FLJ41821 | protéine FJL41821 |
| HGSNAT | héparane-α-glucosaminide N-acétyltransférase |
| HIVEP2 | protéine de liaison d'activateur de type I de VIH 2 |
| HSD11B2 | hydroxystéroïde (11-bêta) déshydrogénase 2 |
| IL8 | interleukine-8 |
| ISG15 | modificateur de type ubiquitine ISG15 |
| KLHL5 | type Kelch 5 (Drosophile) |
| LOC401027 | protéine hypothétique LOC401027 |
| MGC48628 | similaire à la protéine KIAA1680 (= protéine hypothétique LOC401145) |
| MMP2 | matrice métalloprotéinase-2 |
| MYH11 | chaîne lourde de myosine de muscle lisse 11 |
| NOS3 | oxyde nitrique synthase 3 (cellule endothéliale) (eNOS) |
| NR3C1 | récepteur de glucocorticoïdes, sous-famille de récepteurs nucléaires 3, groupe C, membre 1 |
| OGDH | Oxoglutarate (alpha-cétoglutarate) déshydrogénase (lipoamide) |
| OR5C1 | récepteur olfactif, famille 5, sous-famille C, membre 1 |
| PIGK | biosynthèse d'ancrage de phosphatidylinositol glycane, classe K |
| PIK3CD | phosphoinositide-3-kinase, catalytique, polypeptide delta |
| PREB | liaison d'élément régulateur de prolactine |
| RAI16 | acide rétinoïque induit 16 |
| RALGPS1 | Ral GEF avec domaine PH et motif de liaison de SH3 1 |
| RC3H2 | domaines de doigt Ring et de doigt de zinc de type CCCH 2 |
| CDK9 | kinase dépendante de cycline 9 (kinase apparentée à CDC2) |
| CDKN2A | inhibiteur de kinase dépendante de cycline 2A (mélanome, p16, inhibe CDK4) |
| CHRM1 | récepteur cholinergique, muscarinique 1 |
| COMT | catéchol-O-méthyltransférase |
| DRD3 | récepteur de dopamine D3 |
| EGR1 | réponse de croissance précoce 1 |
| FADS1 | désaturase d'acide gras 1 (delta-5 désaturase) |
| FADS2 | désaturase d'acide gras 2 (delta-6 désaturase) |
| FADS3 | désaturase d'acide gras 3 (delta-9 désaturase) |
| FAF1 | facteur 1 associé à Fas (TNFRSF6) |
| FLT1 | tyrosine kinase apparentée à fms 1 (récepteur de facteur de perméabilité vasculaire/VEGF) |
| FOS | homologue d'oncogène viral d'ostéosarcome murin v-fos FBJ |
| FTO | masse graisseuse et obésité associée |
| GABRB1 | récepteur de l'acide gamma-aminobutyrique (GABA) A, bêta 1 |
| GRIK2 | récepteur de glutamate, ionotropique, kainate 2 |
| GRIN3B | récepteur de glutamate, N-méthyl-D-aspartate 3B (sous-unité 3B de récepteur NMDA) |
| HTR2A | récepteur de 5-hydroxytryptamine (sérotonine) 2A |
| IGFBP1 | protéine de liaison de facteur de croissance de type insuline 1 |
| MAOA | monoamine oxydase A |
| MAPK1 | protéine kinase activée par le mitogène 1 (protéine tyrosine kinase ERK2) |
| MYC | homologue d'oncogène viral de myélocytomatose v-myc (aviaire) |
| OTX1 | orthodenticule homéoboîte 1 |
| RXRB | récepteur X des rétinoïdes, bêta |
| S100B | protéine de liaison de calcium S100 B |
| SCD | stéaroyl-CoA désaturase (delta-9 désaturase) |
| SEMA4F | domaine sema, domaine Ig, domaine TM & domaine cytoplasmique court, (sémaphorine) 4F |
| SLC6A3 | transporteur de dopamine dépendant du sodium (transporteur DA) (DAT) |
| SLC6A4 | famille de porteurs de solutés 6 (transporteur de neurotransmetteurs, sérotonine), membre 4 (5-HTT) |

6. Méthode selon l'une quelconque des revendications 2 ou 3 pour la prédiction d'un développement squelettique détérioré associé à un contenu minéral osseux réduit et une propension pour des états pathologiques associés à un contenu minéral osseux bas tels qu'une ostéoporose.

7. Méthode selon la revendication 6, dans laquelle la méthylation du promoteur du gène RXRA en combinaison avec la méthylation du promoteur d'au moins un autre gène est déterminée, dans laquelle l'autre gène est choisi parmi les gènes énumérés dans le Tableau 3 suivant:
**Tableau 3**
| | |
|---|---|
| DEFB107A | défensine, bêta 107 |
| ECHDC2 | domaine d'énoyl-coenzyme A hydratase contenant 2 |
| FLAD1 | homologue de FAD1 flavine adénine dinucléotide synthétase |
| FLJ23577 | protéine KPL2 |
| HGSNAT | héparane-alpha-glucosaminide N-acétyltransférase |
| LOC646870 | protéine hypothétique LOC646870 |
| MCL1 | séquence de leucémie des cellules myéloïdes 1 (apparentée à BCL-2) |
| MMP9 | matrice métallopeptidase 9 (gélatinase B, gélatinase de 92 kDa, collagénase de type IV de 92 kDa) |
| NOS3 | oxyde nitrique synthase 3 (cellule endothéliale) (eNOS) |
| PI3KCD | phosphoinositide-3-kinase, catalytique, polypeptide delta |
| PPARG | récepteur gamma activé par proliférateur de peroxysomes |
| PREB | liaison d'élément régulateur de prolactine |
| RALGPS1 | Ral GEF avec domaine PH et motif de liaison de SH3 1 |
| TNF | facteur de nécrose tumorale (superfamille des TNF, membre 2) (TNFa) |
| TSN | transline |
| VEGFA | facteur de croissance endothéliale vasculaire A |
| CDK9 | kinase dépendante de cycline 9 (kinase apparentée à CDC2) |
| CDKN1A | inhibiteur de kinase dépendante de cycline 1A (p21, Cip1) |
| CDKN2A | inhibiteur de kinase dépendante de cycline 2A (mélanome, p16, inhibe CDK4) |
| CDKN2B | inhibiteur de kinase dépendante de cycline 2B (p15, inhibe CDK4) |
| CDKN2C | inhibiteur de kinase dépendante de cycline 2C (p18, inhibe CDK4) |
| EGR1 | réponse de croissance précoce 1 |
| FADS3 | désaturase d'acide gras 3 (delta-9 désaturase) |
| FAF1 | facteur 1 associé à Fas (TNFRSF6) |
| FLT1 | tyrosine kinase apparentée à fms 1 (récepteur de facteur de perméabilité vasculaire/VEGF) |
| GAD1 | glutamate décarboxylase 1 (cerveau, 67 kDa) |
| GRIK2 | récepteur de glutamate, ionotropique, kainate 2 |
| IGFBP1 | protéine de liaison de facteur de croissance de type insuline 1 |
| MAOA | monoamine oxydase A |
| MYC | homologue d'oncogène viral de myélocytomatose v-myc (aviaire) |
| RXRB | récepteur X des rétinoïdes, bêta |
| SCD | stéaroyl-CoA désaturase (delta-9 désaturase) |
| SEMA4F | domaine sema, domaine Ig, domaine TM & domaine cytoplasmique court, (sémaphorine) 4F |
| SLC6A4 | famille de porteurs de solutés 6 (transporteur de neurotransmetteurs, sérotonine), membre 4 (5-HTT) |

8. Kit comprenant des amorces et des enzymes de restriction sensibles à une méthylation pour une utilisation dans la réalisation d'une amplification sensible à une méthylation d'une combinaison du promoteur du gène RXRA et du promoteur d'au moins un autre gène pour la prédiction d'une ou de plusieurs caractéristiques phénotypiques conformément à la revendication 1, dans lequel l'autre gène est choisi parmi les gènes énumérés dans les Tableaux 2 et 3 des revendications 5 et 7, respectivement.
